(19) Europäisches Patentamt / European Patent Office / Office européen des brevets

(11) **EP 3 842 067 A1**

(12) **EUROPEAN PATENT APPLICATION**

(43) Date of publication:
30.06.2021 Bulletin 2021/26

(51) Int Cl.:
*A61K 39/00* (2006.01)     *A61K 39/39* (2006.01)
*A61P 15/00* (2006.01)

(21) Application number: 20214150.3

(22) Date of filing: 25.07.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(62) Document number(s) of the earlier application(s) in
accordance with Art. 76 EPC:
14898255.6 / 3 191 119

(71) Applicant: **United Biomedical, Inc.**
**Hauppauge, New York 11788 (US)**

(72) Inventors:
• **WANG, Chang Yi**
**Cold Spring Harbor, New York 11788 (US)**

• **PENG, Wen-Jiun**
**Taoyuan (TW)**

(74) Representative: **Böhm, Brigitte**
**Weickmann & Weickmann**
**Patent- und Rechtsanwälte PartmbB**
**Postfach 860 820**
**81635 München (DE)**

Remarks:
This application was filed on 15-12-2020 as a
divisional application to the application mentioned
under INID code 62.

(54) **IMMUNOGENIC LHRH COMPOSITION AND USE THEREOF IN PIGS**

(57)    A vaccine composition for castrating pigs, comprising a peptide immunogen and a veterinarily acceptable delivery vehicle or adjuvant, wherein the peptide immunogen comprises (a) a LHRH peptide of SEQ ID NO: 1, and (b) at least one T helper epitope selected from a group consisting of SEQ ID NOs: 2, 3, 4, and 5, and, optionally, an immunostimulatory peptide of SEQ IN NO: 6, wherein the LHRH peptide is covalently linked through its N-terminus residue to the T helper epitope or immunostimulatory peptide. A method for castrating or inhibiting characteristics, including boar taint, induced by the sexual maturation of pigs using the vaccine composition is also disclosed.

EP 3 842 067 A1

**Description**

**FIELD OF THE INVENTION**

The present invention relates to an immunogenic LHRH composition, and in particular relates to an immunogenic LHRH composition containing LHRH comprising peptide constructs that leads to functional suppression of LHRH level in pigs resulting in effective immunocastration, removal of boar taint and growth enhancement in pigs.

**BACKGROUND OF THE INVENTION**

Gonadotropin-releasing hormone (GnRH), also known as Luteinizing-hormone-releasing hormone (LHRH), is a trophic peptide hormone responsible for the release of follicle-stimulating hormone (FSH) and luteinizing hormone (LH) from the anterior pituitary. LHRH is synthesized and released from neurons within the hypothalamus.

Vaccination against the hypothalamic hormone Luteinizing-hormone-releasing-hormone (LHRH) has been demonstrated as an immunological method of controlling reproduction since the early 1970's. Eliciting an immune response to LHRH prevents the release from the anterior pituitary of the hormones LH and FSH, which are required for the development and maintenance of the gonads - the testes in the male and ovaries in the female. Thus reduction of LH and FSH levels leads to loss of reproductive function.

De-sexing (or neutering) operations are the most widely practiced surgical procedures in veterinary medicine and livestock animal management. A significant proportion of both sexes of domestic livestock and companion animals are routinely surgically de-sexed to prevent a variety of undesirable characteristics which accompany sexual maturity.

Immunological blocking of LHRH action can result in infertility in animals because LHRH controls testosterone production, which, in turn regulates the development of sperm and estrogen production, in turn causing the ripening of ova. Moreover, LHRH-based immunotherapy provides a means for contraception in male and female companion animals (e.g. dogs, cats, horses and rabbits) as well as mitigating undesirable androgen-driven behavior such as heat, territorial marking and aggression. Such process is reversible depending on the level of serum antibodies in treated animals upon immunization with LHRH- based immunotherapy. Lastly, immunological castration (e.g. antibody-based inhibition of LHRH action) has an additional application in the meat animal industry. Males are not processed into prime cuts of meat because of the offensive aroma and taste associated with their flesh as a result of circulating testosterone (e.g. boar taint). Since mechanical (e.g. surgical) castration of male food animals is no longer considered humane, immunological castration provides an acceptable alternative to this practice.

Several immunogenic forms of LHRH have been tested. For example, LHRH peptide has been conjugated to carrier protein(s) to enhance the peptide hormone's immunopotency. However, these protein carriers are too expensive for large scale use and the resultant peptide-protein conjugates are not as effective to (1) yield immunocastration over a long duration nor are they able to (2) generate anti-LHRH immune response in all animals, both conditions are required for an efficacious vaccine as a replacement for surgical castration.

Further, effective immunization with LHRH, a non-immunogenic 10-mer peptide, depends on the conjugation site between LHRH and the carrier protein(s). Moreover, protein linkage to LHRH is problematic as an immunogen because the majority of immune responses toward such an immunogen are directed to the large carrier protein(s) rather than to the LHRH peptide (the mass of the toxins or other carrier proteins are far larger than that of LHRH, a 10-mer peptide). This phenomenon frequently leads to carrier protein-induced epitopic immune suppression. Accordingly, an immune enhancer that is suitable for linkage to the LHRH peptide yielding inexpensive peptide construct for use as the key ingredient in a vaccine formulation capable of stimulating an early and strong immune response to the master hormone LHRH for multiple applications in immunocastration shall be sought. Likewise this immune enhancer also should avoid carrier-induced epitopic suppression. Therefore, an LHRH vaccine which has consistently high efficacy for immunocastration including (1) specific applications for removal of boar taint along with enhanced growth profile in pigs; and (2) for behavior modification to allow ease in animal management in cattle; is still highly desired to resolve current deficiencies in LHRH based immunocastration, in particular in pigs.

**SUMMARY OF THE INVENTION**

The invention provides a vaccine composition for pig castration, comprising a peptide immunogen and a veterinarily acceptable delivery vehicle or adjuvant, wherein the peptide immunogen comprises (a) an LHRH peptide of SEQ ID NO: 1, and (b) at least one T helper epitope or an immunostimulatory element selected from a group consisting of SEQ ID NOs: 2, 3, 4, 5, and 6, wherein the peptide of LHRH can be covalently linked through its N-terminus with the T helper epitope and/or an immunostimulatory element by a spacer sequence of Gly-Gly or εNLys. The spacer "εNLys" is a lysine residue present between two amino acids that is bound to (1) the C-terminus of the preceding amino acid at the ε-NH$_2$ group of the lysine residue and (2) the N-terminus of the following amino acid at the C-terminus of the lysine residue. The terms "εNLys" and "εLys" and "εK" can be used interchangeably.

In another embodiment, the peptide immunogen of the present invention comprises SEQ ID NOs: 7, 8, 9, or 10, or a mixture thereof, and the veterinarily acceptable adjuvant is selected from a group consisting of ISA50V2 and Emulsigen D. The total amount of the peptide immunogen is more than 6.25 μg per dose, preferably, 50 μg to 200 μg.

Another aspect of the present invention relates to a method for inhibiting characteristics induced by the sexual maturation of pigs, comprising administering an effective amount of a vaccine composition of the present invention to pigs to reduce the production of testosterone and its derivatives such as dihydrotestosterone and estrogen in the immunized host. The characteristics include, but are not limited to, boar taint, sexual activity, fertility, and estrous behavior. The method of the present invention inhibits boar taint and the growth of testes or epididymides.

In one embodiment, the vaccine composition is administered by intramuscular injection into male pigs in a two-dose series with the first dose of the vaccine composition being applied to the pig at as early as 3 weeks of age with the second shot as a boost from 10 to 16 weeks or older, leading to effective immunocastration, enhanced growth, and removal of boar taint about two weeks after the boost.

Detailed description of the invention is given in the following embodiments with reference to the accompanying drawings.

## References:

(1) Improvac scientific discussion from European Medicines Agency (EMA) website (last updated on 2010/04/22): http://www.emea.europa.eu/docs/en_GB/document_library/EPAR_-_Scientific_Discussion/veterinary/000136/WC500064057.pdf

(2) Wang, CY; Zamb, TJ.; YE, John; Kaminsky, SM.; Hosein, Barbara; Nixon, DF.; Koff, CW; Kowalski, Jacek; Walfield, A M., Structured synthetic antigen libraries as diagnostics, vaccines and therapeutics. WO 95/11998. United States: United Biomedical Inc.

(3) Anna Efim Ladd, Chang Yi Wang, Timothy Joseph Zamb., "Immunogenic LHRH peptide constructs and synthetic universal immunes stimulators for vaccines", US Patent No. 5,759,551. United States: United Biomedical Inc.

(4) Wang, CY. "Artificial T helper cell epitopes as immune stimulators for synthetic peptide immunogens including immunogenic LHRH peptides", US Patent Nos. 6,025,468, 6,228,987, 6,559,282. United States: United Biomedical, Inc.

(5) Wang CY. Artificial T helper cell epitopes as immune stimulators for synthetic peptide immunogens. US Patent Nos. 6,713,301. United States: United Biomedical Inc.

(6) Meister, et al., "Two novel T cell epitope prediction algorithms based on MHC-binding motifs; comparison of predicted and published epitopes from Mycobacterium tuberculosis and HIV protein sequences", Vaccine, 13(6):581-591.

(7) Synthetic Peptides: A Users Guide. Grant GA, ed. New York: WH Freeman and Company: 1992

## BRIEF DESCRIPTION OF THE DRAWINGS

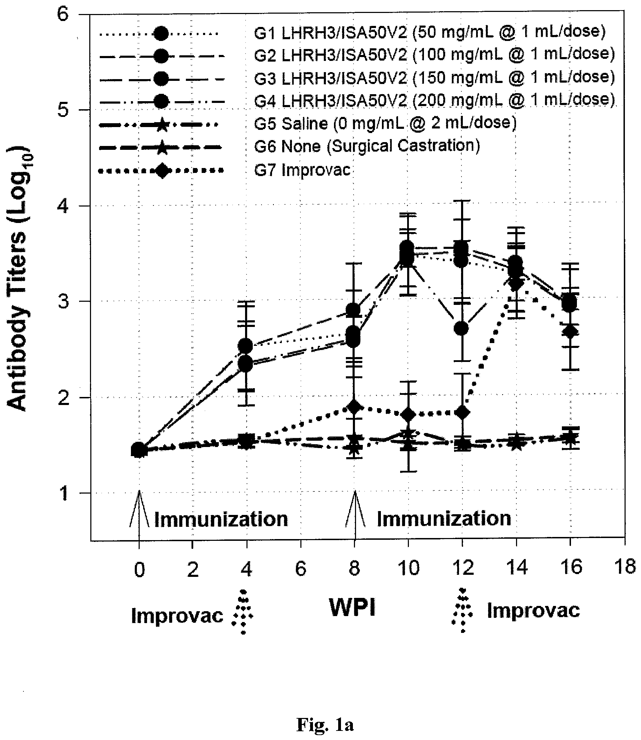

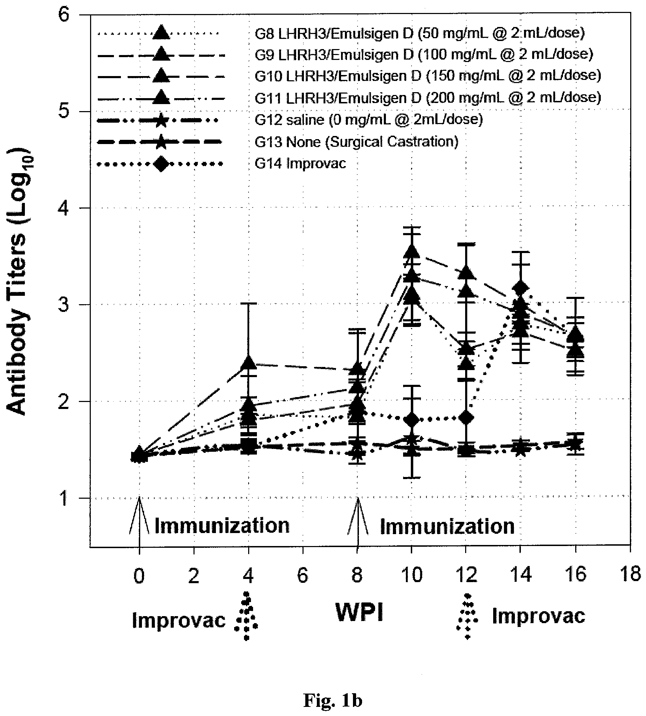

**Figures 1a and 1b** illustrate the respective anti-LHRH antibody titers after immunization of male pigs with formulations containing LHRH3 (SEQ ID NOs: 7, 8 and 9) in adjuvant ISA50V2 (1a) or Emulsigen D (1b). Pigs in all testing groups (Groups 1 to 4 and group 8 to 11) have a high titer of antibodies against LHRH two weeks after the boost (i.e. 10 weeks post the initial immunization, or 10 WPI, which is about 18 weeks of age). In comparison, the LHRH-antibody titers in pigs receiving the currently available commercial vaccine Improvac® (Group 7 and group 14) only rise at 14 WPI with titers lower than those of the peptide vaccine formulations of the current invention (Groups 1-4 and group 8-11). Pigs in Groups 5 and 12 received saline and served as negative controls and pigs in groups 6 and 13 were castrated by surgical procedure and served as positive controls.

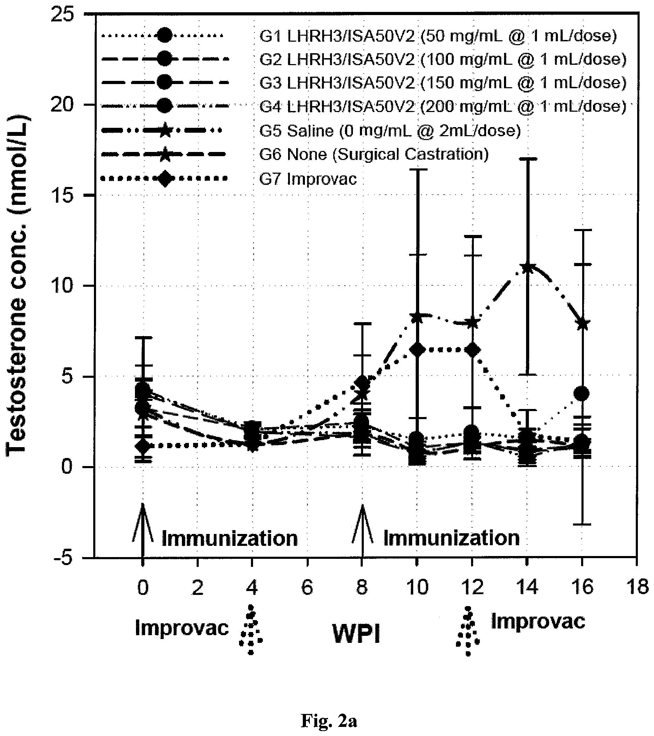

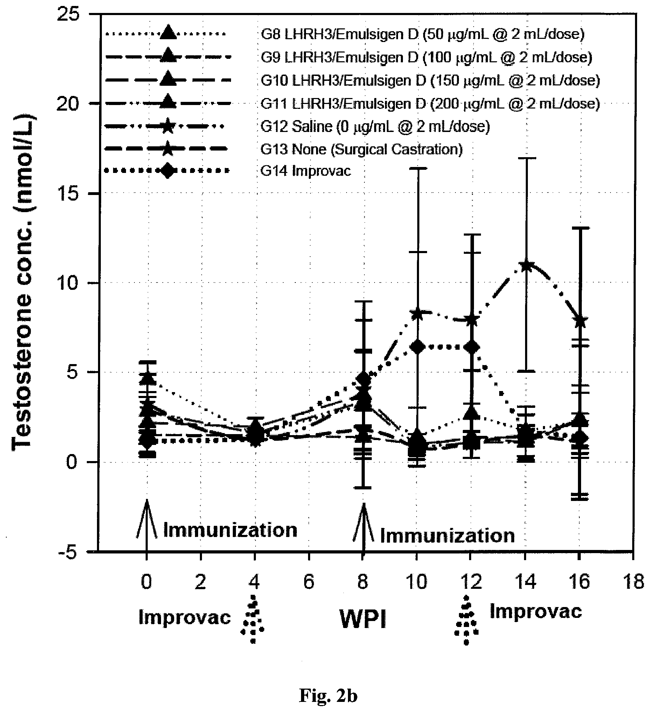

**Figures 2a and 2b** illustrate the castration duration of the respective animals by illustrating the corresponding testosterone concentrations in pigs after immunization of male pigs with formulations containing LHRH3 (SEQ ID NOs: 7, 8 and 9) in formulations containing adjuvant ISA50V2 (2a) or Emulsigen D (2b). All groups formulated in ISA50V2 or Emulsigen D maintained a low level of testosterone by 10 WPI and remained low until 16 WPI (24 weeks old). In comparison, pigs in Groups 7 and 14 receiving Improvac® vaccine had high levels of serum testosterone until 12 WPI and which low level began to climb up beginning at 14 WPI, indicating a far lower efficiency in immunocastration of Improvac®.

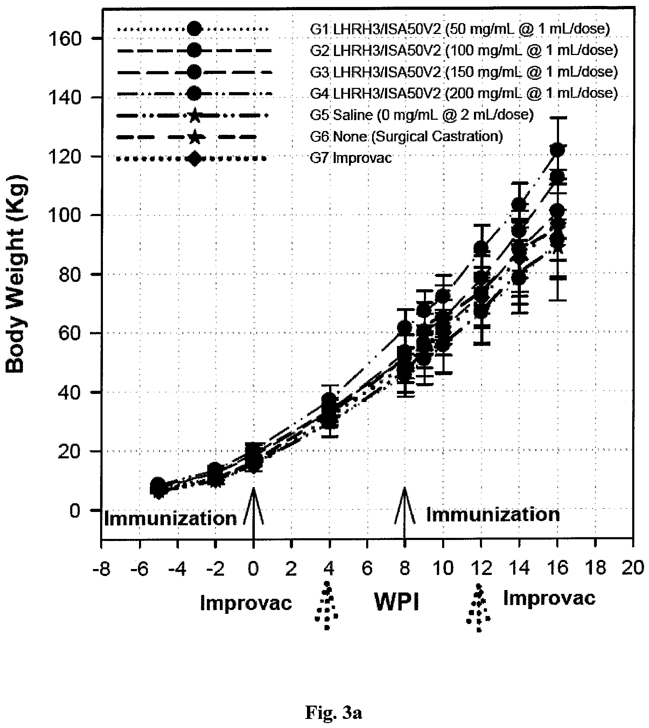

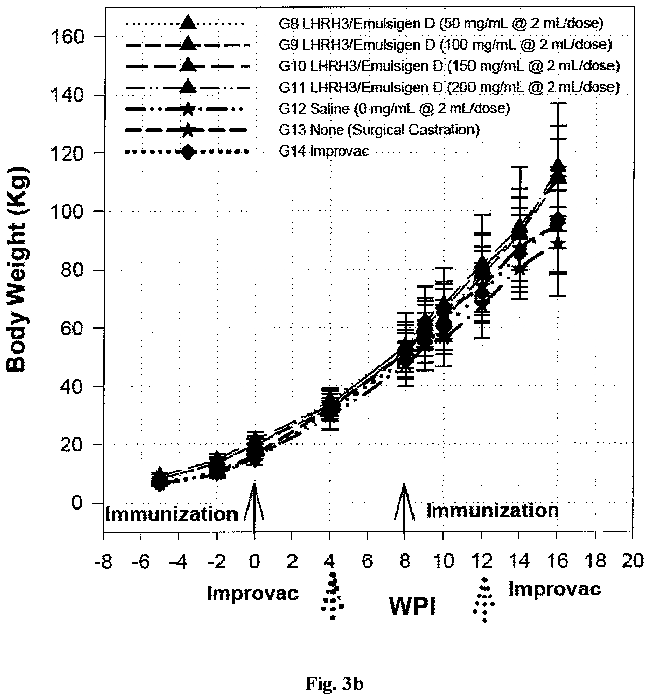

**Figures 3a and 3b** illustrate the respective body weights of pigs at the age of 24 weeks after immunization of male pigs with formulations containing LHRH3 (SEQ ID NOs: 7, 8 and 9) in formulations containing adjuvant ISA50V2 (3a) or Emulsigen D (3b). The nonimmunized negative control and the surgically castrated groups had significantly lower average body weights of 94.9 kg (negative controls, Groups 5 and 12) and 88.8 kg (castrated groups 6 and 13). In comparison to the surgically castrated groups, the vaccine of the current invention (Groups 4 and 11) demonstrated a significantly greater benefit in body weight gain by about 27.9%.

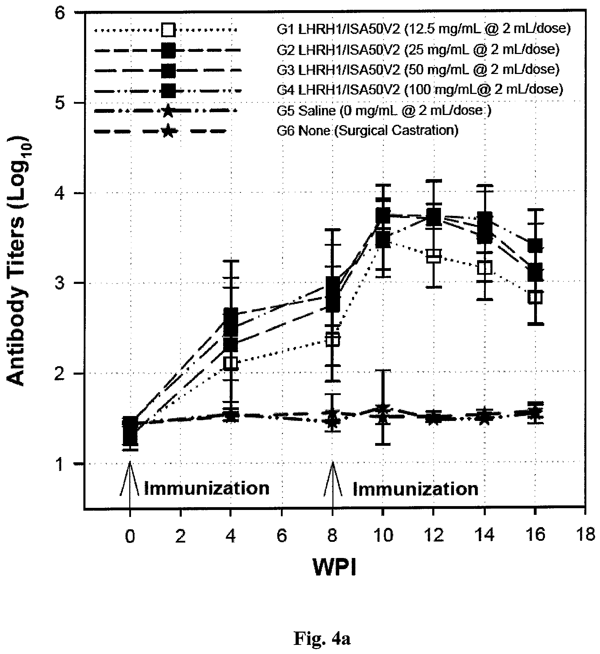

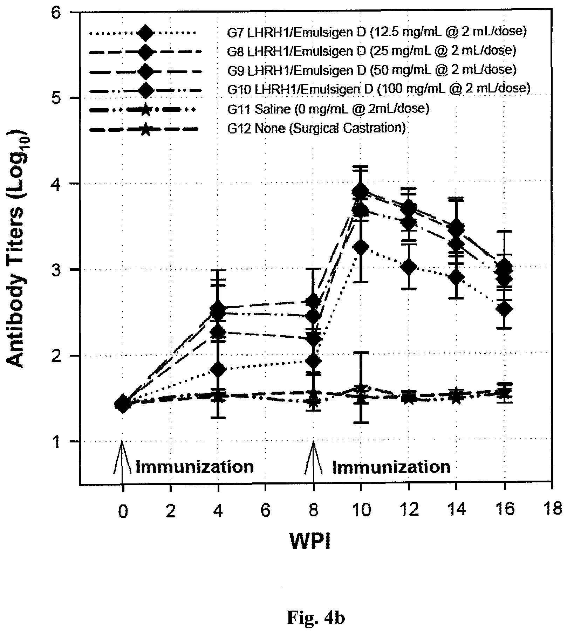

**Figures 4a and 4b** illustrate the respective anti-LHRH antibody titers after immunization of male pigs with formulations containing LHRH1 (SEQ ID NO: 10) in adjuvant ISA50V2 (4a) or Emulsigen D (4b). Pigs in all testing groups had high titers of antibodies against LHRH after two weeks of boost at 10 WPI (i.e. 18 weeks of age). The antibody titers could be maintained near 3.0 ($Log_{10}$) until 16 WPI (i.e. 24 weeks of age) when the pigs are for sale on the markets for both groups formulated in either ISA50V2 or in Emulsigen D.

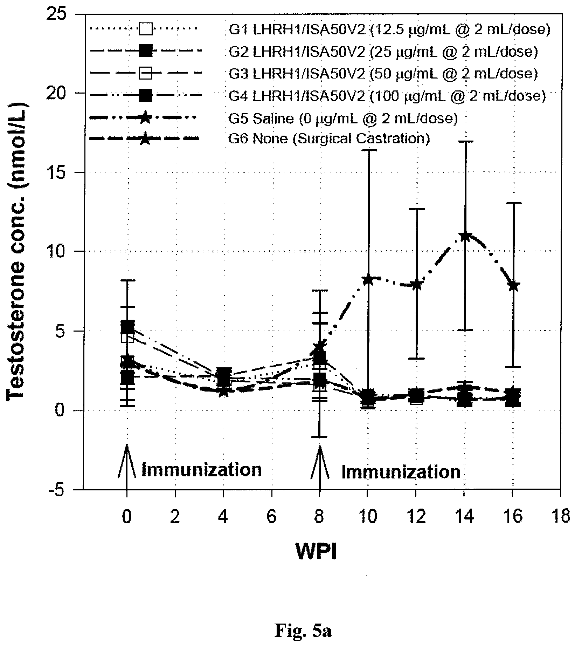

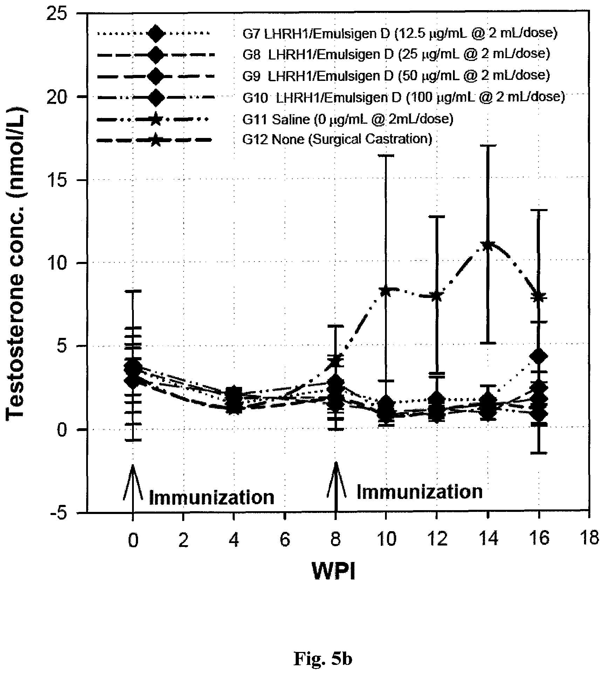

**Figures 5a and 5b** illustrate the respective testosterone concentrations after immunization of male pigs with formulations containing LHRH1 (SEQ ID NO: 10) in adjuvant ISA50V2 (5a) or Emulsigen D (5b). Serum testosterone concentrations in pigs from Groups 1 to 4 formulated in ISA50V and Groups 8 to 11 formulated in Emulsigen D correspond reversely to the serum titers of LHRH-antibodies, i.e. the higher anti-LHRH antibody titers, the lower serum testosterone concentration until achieving the castrating serum testosterone level with a cut-off value estimated at 1.87 nmol/L at two weeks after the boost (i.e. 10 WPI or18 weeks of age). All groups, except for group 1, formulated in ISA50V2 or Emulsigen D maintained a low level of serum testosterone concentration until 16 WPI (24 weeks of age).

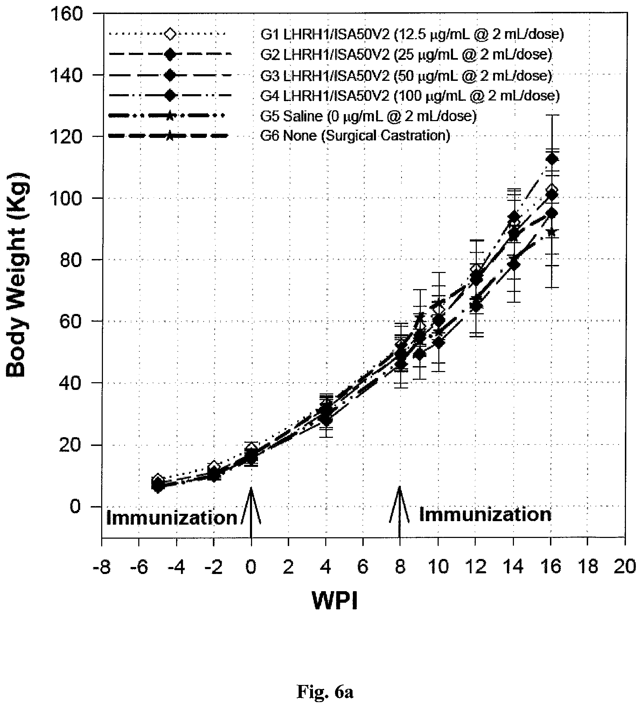

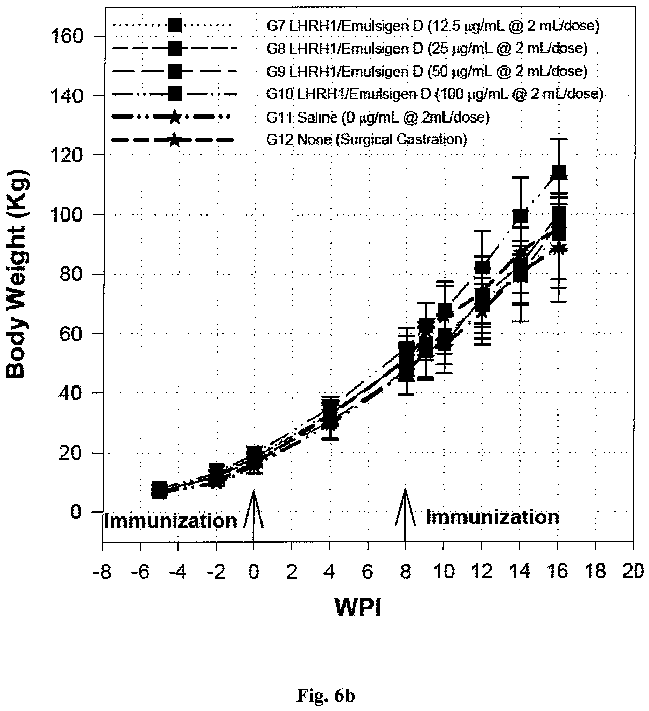

**Figures 6a and 6b** illustrate the respective body weights of pigs after immunization of male pigs with formulations containing LHRH1 (SEQ ID NO: 10) in adjuvant ISA50V2 (6a) or Emulsigen D. The negative control, i.e. non-vaccinated, groups (Groups 5 and 11) and the surgically castrated groups (Groups 6 and 12) had significantly lower average body weights of 94.9 kg and 88.8 kg respectively at the age of 24 weeks. In comparison to the pigs in the surgically castrated groups, the vaccine formulations of the current invention (Groups 4 and 10) had a significantly greater benefit in body weight gain by about 20.3%.

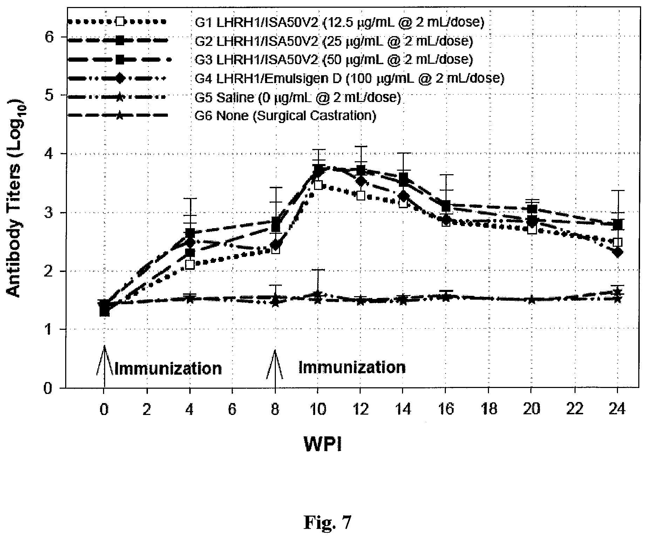

**Figure 7** illustrates the duration of anti-LHRH antibody titers after immunization of male pigs with formulations containing LHRH1 (SEQ ID NO: 10) in adjuvant ISA50V2 or Emulsigen D. Two weeks after the boost (i.e. 10 WPI), anti-LHRH antibody titers of Groups 1 to 4 reached more than 3.0 ($log_{10}$) and such high titers were sustained till 20 WPI. The immunized animals of Groups 2 and 3 could even maintain the anti-LHRH antibody titers at 3.0 ($log_{10}$) until 24 WPI (i.e. 32 weeks of age). In contrast, the negative control (Group 5) and surgical castration control (Group 6) always kept a low titer of anti LHRH antibodies.

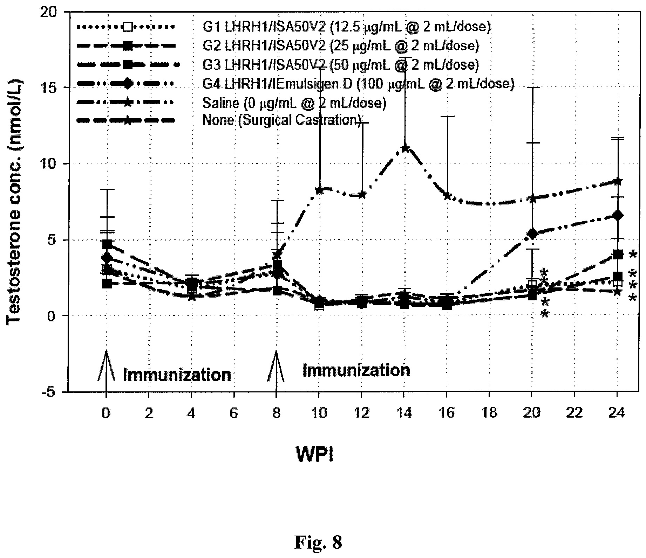

**Figure 8** illustrates the castration duration of the respective testosterone concentrations in pigs after immunization of male pigs with formulations containing LHRH1 (SEQ ID NO: 10) in adjuvant ISA50V2 or Emulsigen D. Two weeks after the boost (i.e. 10 WPI), a low level of serum testosterone was found in all pigs in Groups 1 to 4 and reached the castration level by suppression of anti-LHRH antibodies demonstrated in these groups. The testosterone level of pigs in Groups 1 to 4 maintained as low as that of the castration control group (Group 6) to 20 WPI (i.e. 28 weeks of age).

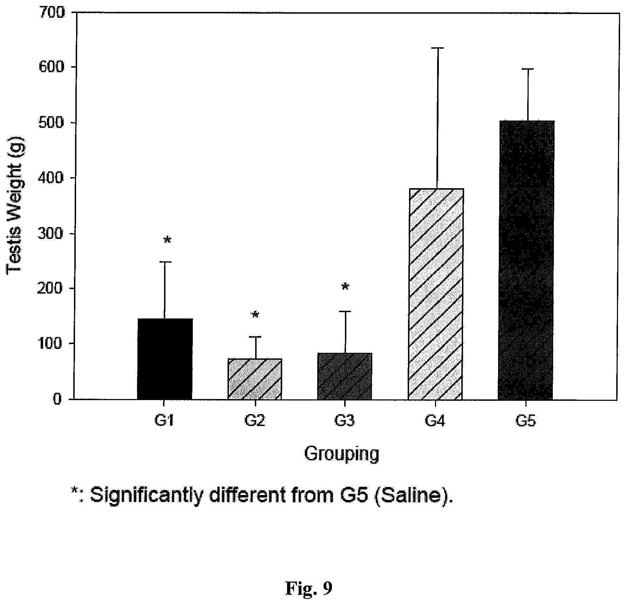

**Figure 9** illustrates the testes weight of pigs after immunization with formulations containing LHRH1 (SEQ ID NO: 10) in adjuvant ISA50V2 or Emulsigen D at 24 WPI. In Groups 1 to 4, the testes of the pigs shrank to nonfunctional size.

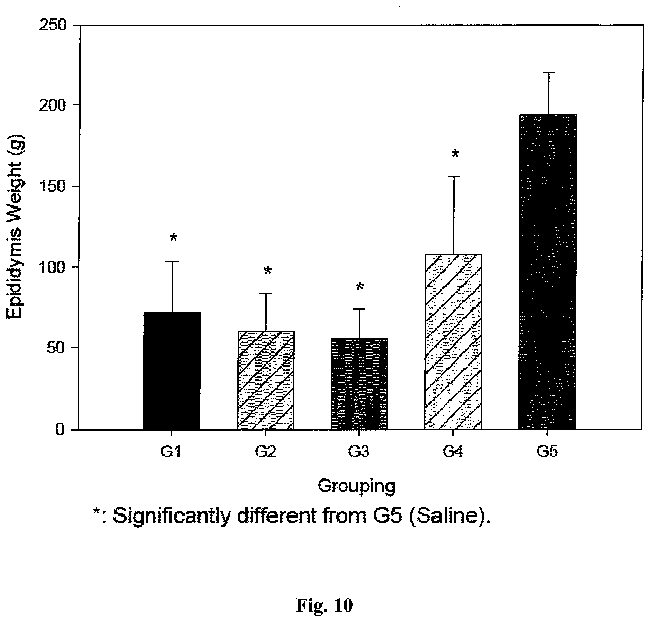

**Figure 10** illustrates the epididymides weight of pigs after immunization with formulations containing LHRH1 (SEQ ID NO: 10) in adjuvant ISA50V2 or Emulsigen D at 24 WPI. In Groups 1 to 4, the weight of epididymides was significantly decreased.

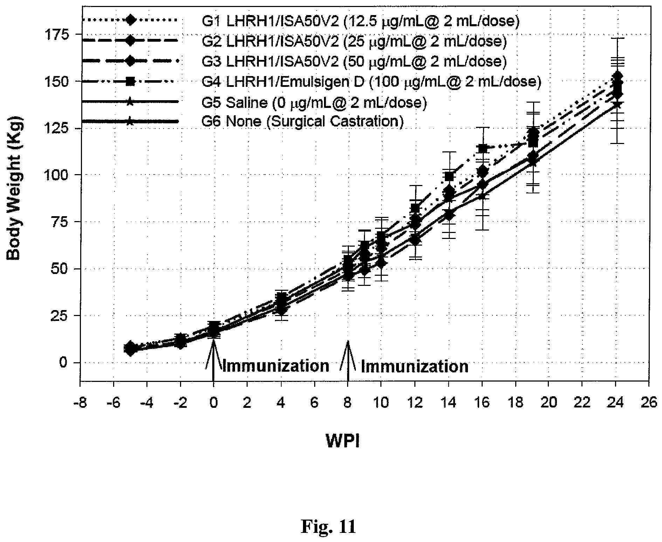

**Figure 11** illustrates the body weight of pigs after immunization with formulations containing LHRH1 (SEQ ID NO:

10) in adjuvant ISA50V2 or Emulsigen D. The immunized animals of Groups 1 to 4 have a far better body weight gain performance than the negative control and the surgically castrated control groups (Groups 5 and 6).

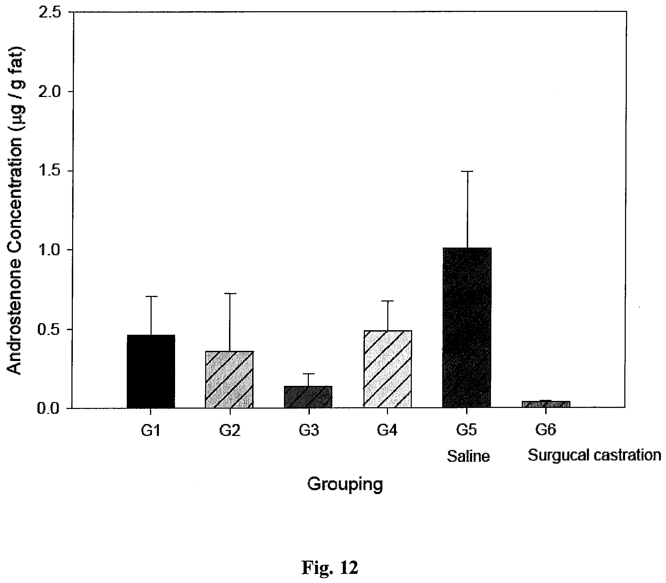

**Figure 12** illustrates the effect over androstenone concentration of pigs after immunization with LHRH1 (SEQ ID NO: 10) in adjuvant ISA50V2 or Emulsigen D. The concentration of androstenone in fat was controlled below 0.5 $\mu$g/g fat in Groups 1 to 4. For Group 3, the mean value of androstenone was even controlled at 0.1384 $\mu$g/g fat.

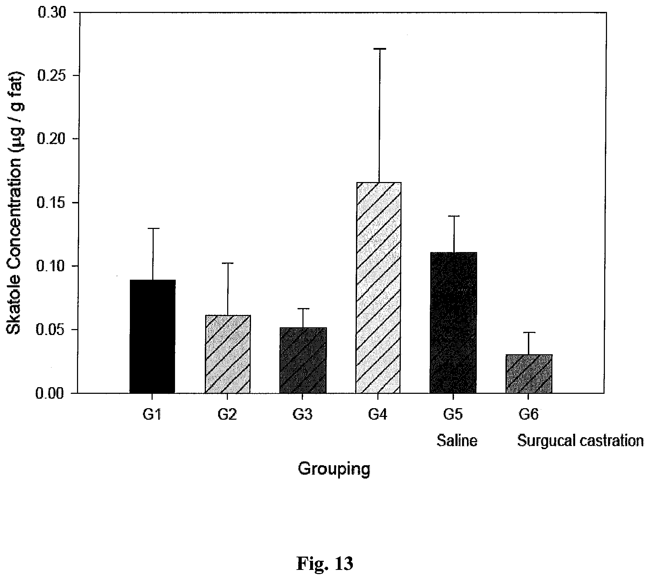

**Figure 13** illustrates the effect over skatole concentration of pigs after immunization with LHRH1 (SEQ ID NO: 10) with adjuvant ISA50V2 or Emulsigen D. The concentration of skatole was controlled under 0.1 $\mu$g/g fat in Groups 1 to 3. In Group 3, the mean value of skatole was found the lowest (0.0514 $\mu$g/g).

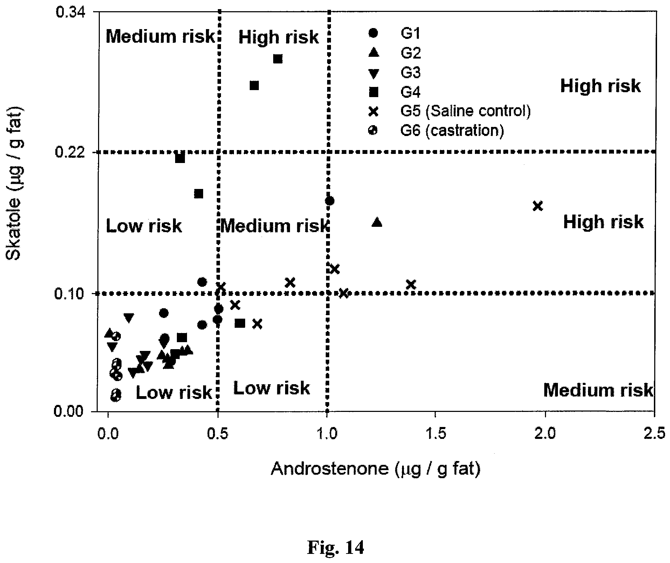

**Figure 14** illustrates a boar taint factor distribution plot. Group 1 had a similar performance as Group 2 in which 7/8 of the samples distributed in the low risk section with one sample in the medium/high risk section. Group 3 had excellent performance until 24 WPI in which all of the samples were distributed in the low risk section. Surgical castration group (Group 6) always kept the lowest concentration of boar-taint factors. Saline control group (Group 5) had just 2/8 in the low risk section, 2/8 in the medium risk section and 4/8 in the high risk section.

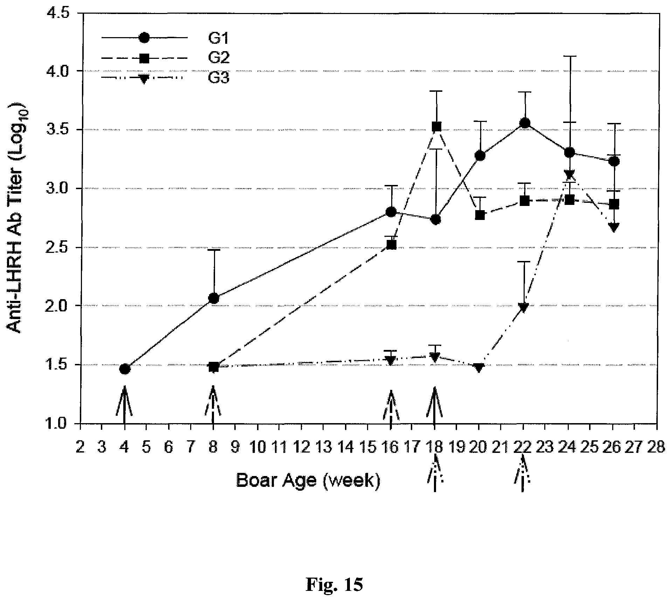

**Figure 15** illustrates anti-LHRH antibody titers after immunization of male pigs with formulations containing LHRH1 (SEQ ID NO: 10) in adjuvant ISA50V2 with various prime and boost schedules for assessment of flexibility in immunocastration schedule. Pigs in group 1 demonstrated a high immunogenicity with LHRH antibody mean titers up to 3.556 ($\log_{10}$) at 22 weeks of age and maintained at 3.235 ($\log_{10}$) at 26 weeks of age. Group 2 achieved its highest antibody titer with a mean titer at 3.526 ($\log_{10}$) at 18 weeks of age and maintained at 2.869 ($\log_{10}$) at 26 weeks of age. Group 3 achieved its highest mean antibody titer of at 3.219 ($\log_{10}$) at 24 weeks of age and maintained at 2.682 ($\log_{10}$) at 26 weeks of age.

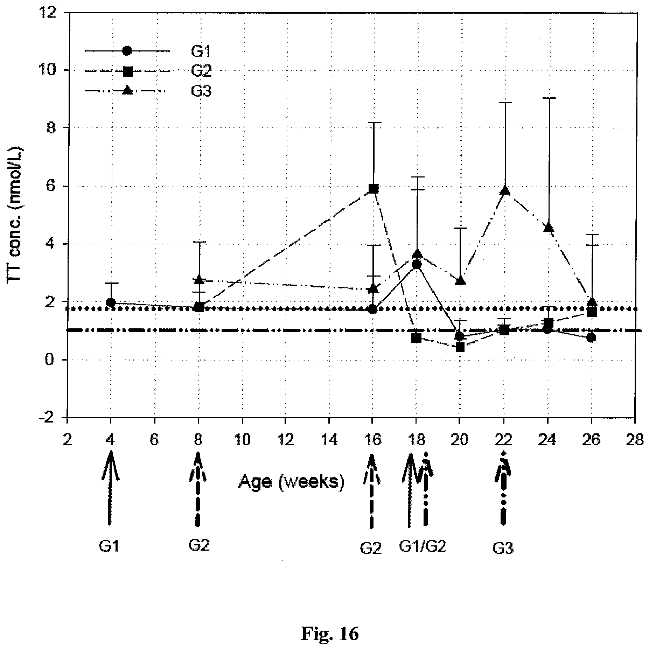

**Figure 16** illustrates the serum testosterone concentrations after immunization of male pigs with formulations containing LHRH (SEQ ID NO: 10) in adjuvant ISA50V2 with various prime and boost schedules for assessment of flexibility in immunocastration schedule. Pigs in group 1 show an immunocastration effect (testosterone concentration ≦ 1.87 nmol/L) at 20 weeks of age, i.e. 2 weeks post the boost and such effect was sustained at least 6 weeks until 26 weeks of age. The testosterone concentrations of pigs in Group 2 was significantly lower than 1.87 nmol/L during the 2 to 6 weeks period after the boost (i.e. from 22 weeks to 26 weeks of age) and remained low (testosterone concentration ≦ 1.644 nmol/L) until 26 weeks of age. Two weeks after the boost, the mean testosterone concentration of Group 3 was 4.535 nmol/L.

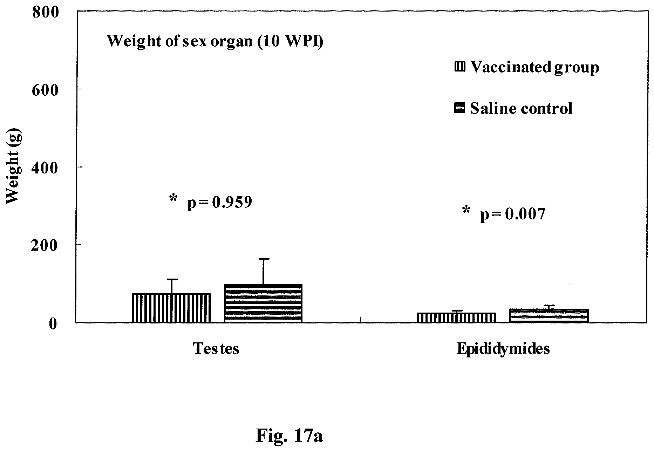

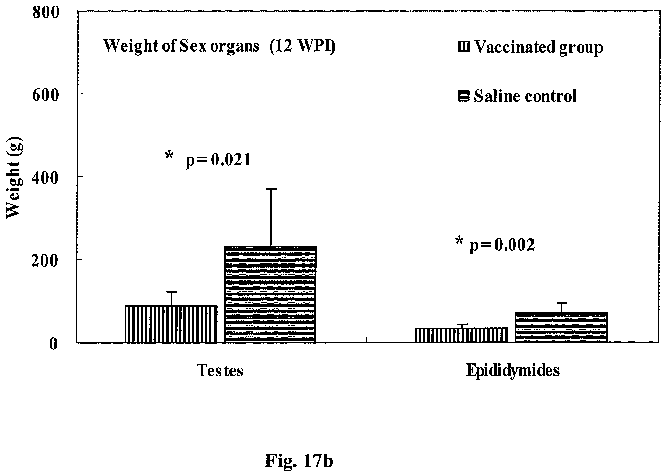

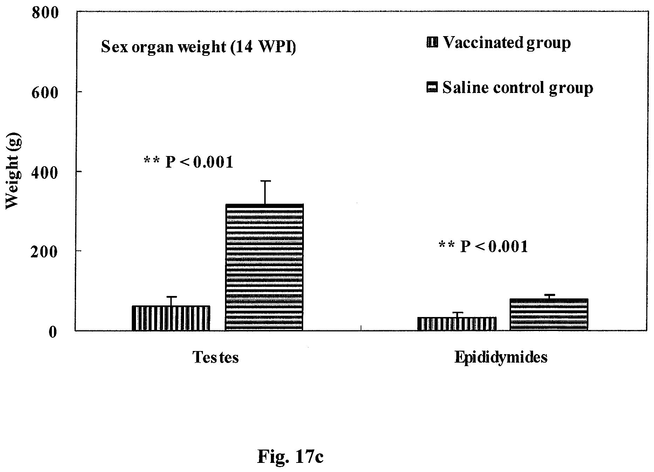

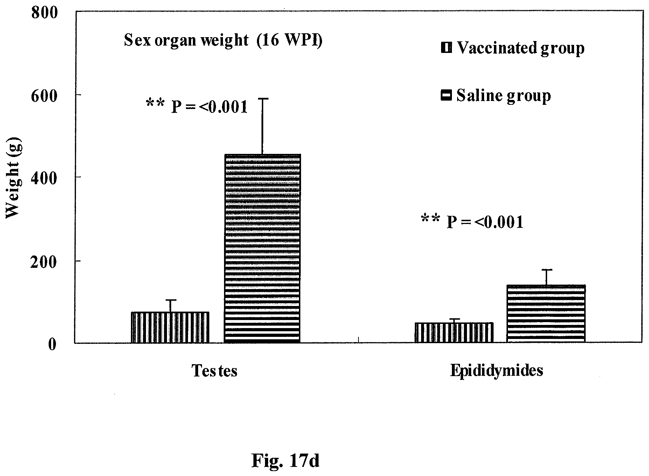

**Figure 17a, 17b, 17c, and 17d** illustrate the weight of testes and epididymides in pigs after immunization with formulations containing LHRH3 (SEQ ID NOs: 7, 8 and 9) in adjuvant ISA50V2. The weight of the testes was measured at 10 WPI **(17a)**, 12 WPI **(17b)**, 14 WPI **(17c)**, and 16 WPI **(17d)**. The testes and epididymides shrank gradually over time and had a significant weight loss at 10 WPI for epididymides and at 12 WPI (p < 0.001) for testes.

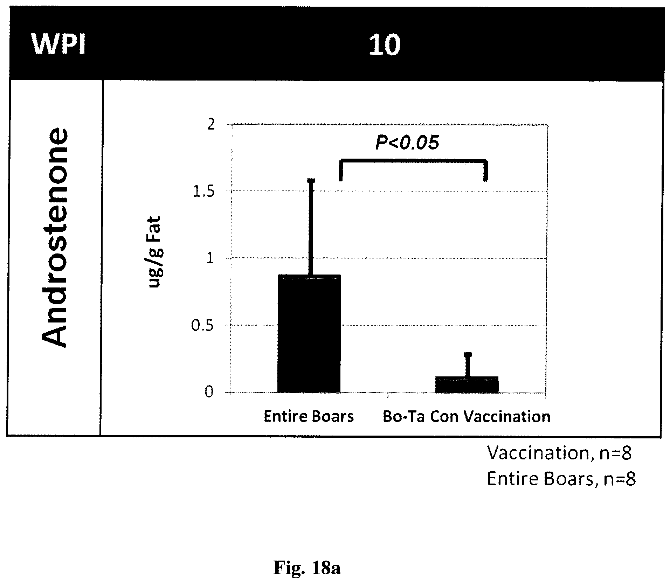

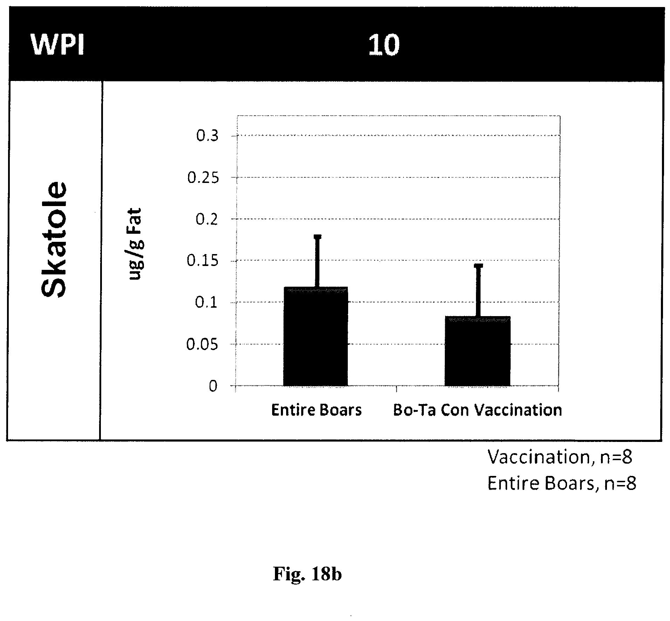

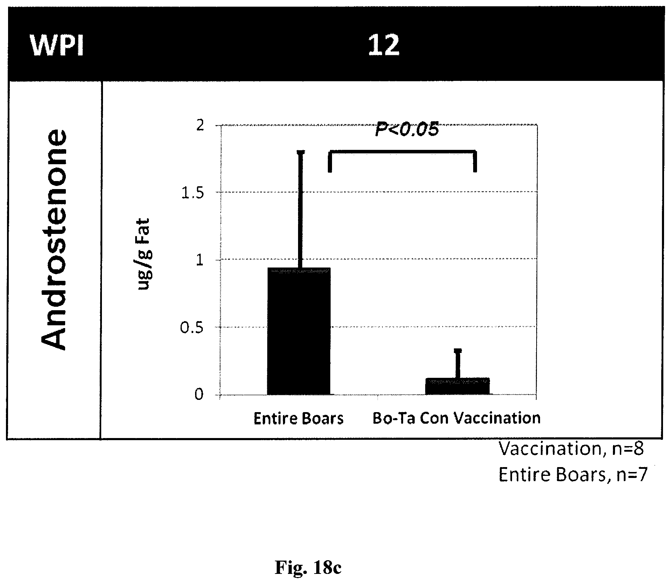

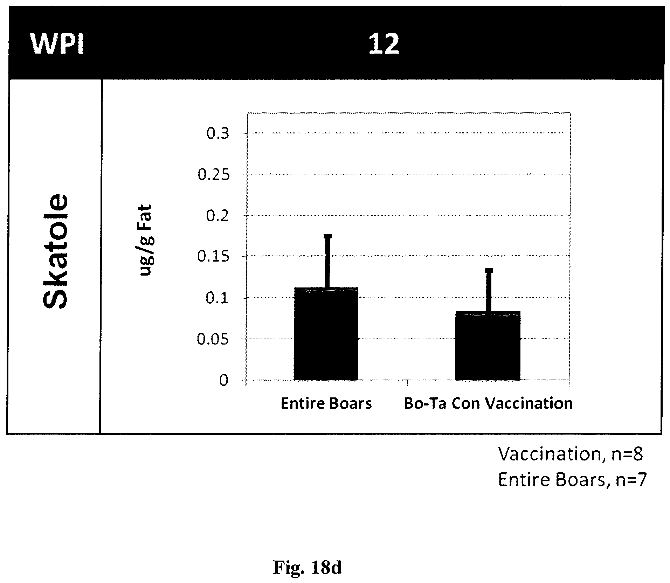

**Figure 18a, 18b, 18c, and 18d** illustrate the amount of boar taint factor (androstenone and skatole) after immunization of male pigs with formulations containing LHRH3 (SEQ ID NOs: 7, 8 and 9) in adjuvant ISA50V2. The amount of androstenone and skatole was evaluated at 10 WPI **(18a** and **18c,** respectively) and 12 WPI **(18b** and **18d,** respectively). After immunization, the testosterone was suppressed to castration level (i.e. lower than 1.87 nmol/L) resulting in the decrease of the androstenone in fat.

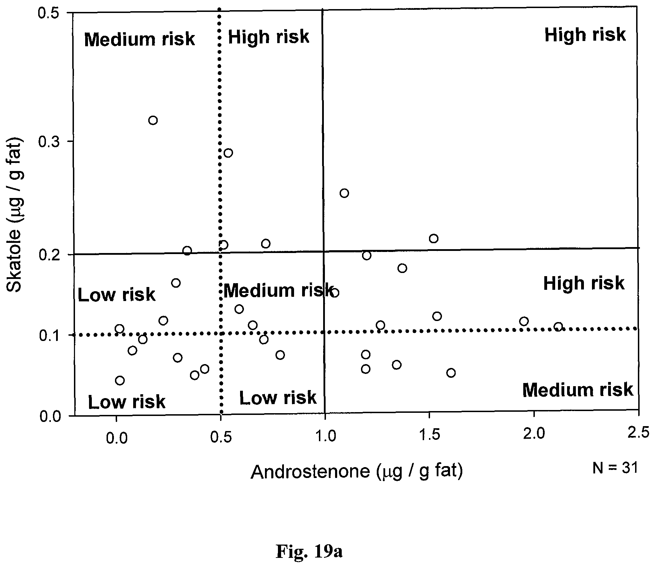

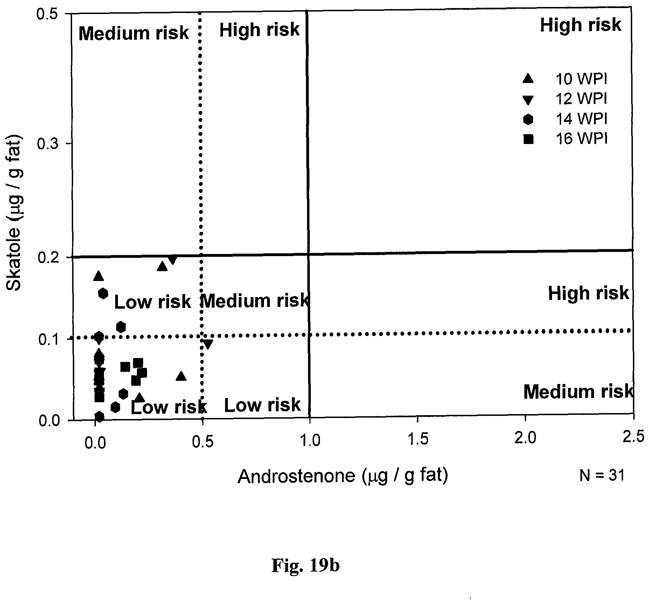

**Figure 19a and 19b** illustrate the risk evaluation through results of androstenone and skatole level in fat in a negative control group of male pigs that did not receive treatment **(19a)** compared to male pigs that were immunized with LHRH peptide vaccine formulations **(19b).** All of the vaccinated groups were found in the low risk section of sensitized assessment (low androstenone (< 0.5 $\mu$g/g fat) and low skatole concentration (< 0.22 $\mu$g/g fat)) from 10 WPI to 16 WPI.

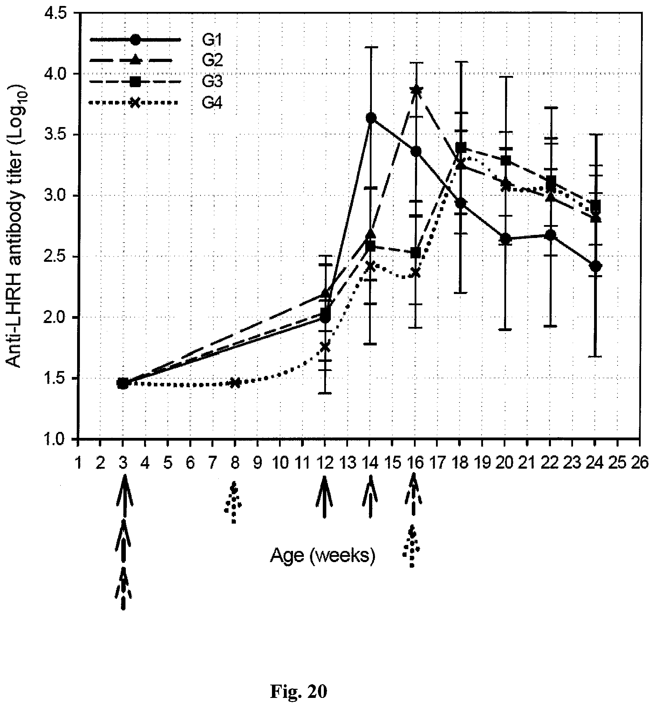

**Figure 20** illustrates the duration effect of anti-LHRH antibody titers after immunization of male pigs with formulations containing LHRH1 (SEQ ID NO: 10) in adjuvant ISA50V2. The antibody titer of Group 1 declined from 3.636 ± 0.577 (Log10) (at the peak) to 2.418 ± 0.742 (Log10) (at the end of the study). The antibody titer of Group 2 declined from 3.868 ± 0.221 (Log10) to 2.806 ± 0.213 (Log10) (at the end of the study).

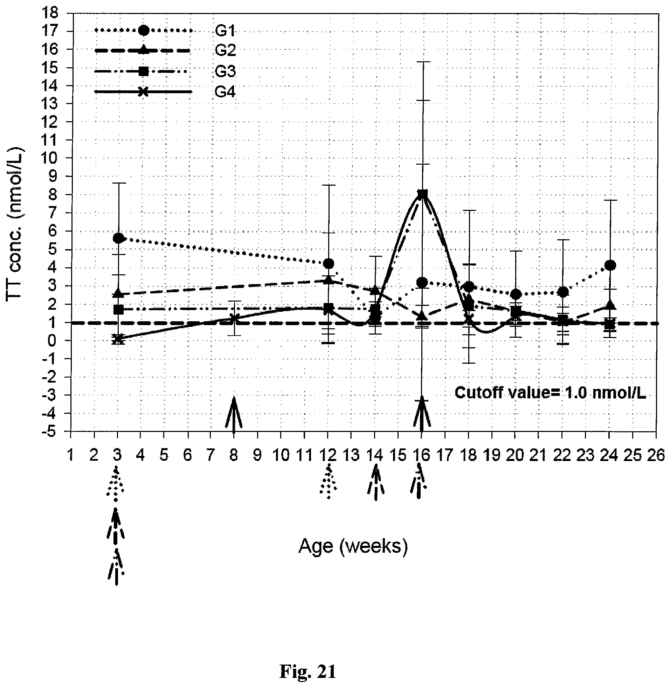

**Figure 21** illustrates the testosterone concentration after immunization of male pigs with formulations containing LHRH1 (SEQ ID NO: 10) in adjuvant ISA50V2. The testosterone concentration of all the groups at the initial stage of the study was 2.943 ± 2.854 (mean ± SD) nmol/L. The time point set for vaccine potency evaluation was at 2 weeks post boost, which showed the lowest testosterone concentration. The average testosterone concentrations of pigs in groups 1 to 4 at 2 weeks post boost were 1.265 ± 0.353, 1.329 ± 0.636, 1.904 ± 2.297 and 1.222 ± 0.445 (mean ± SD) nmol/L, respectively.

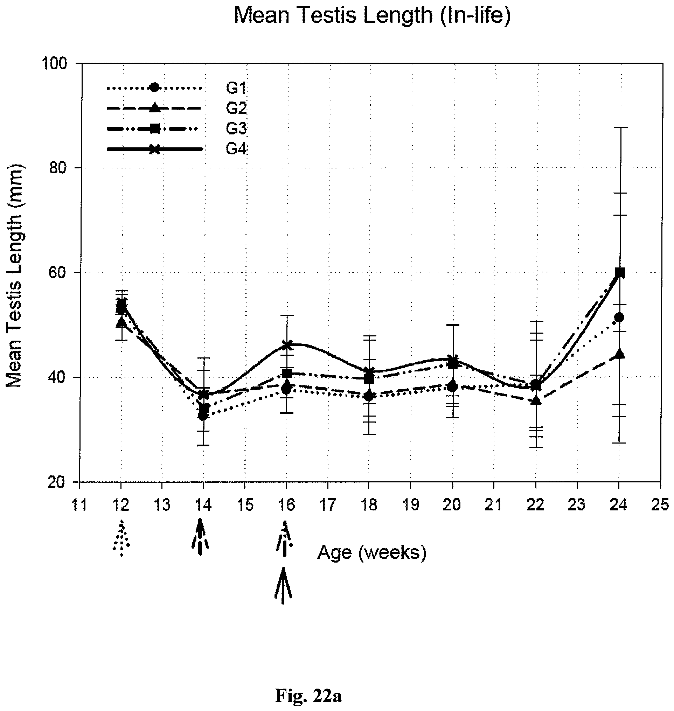

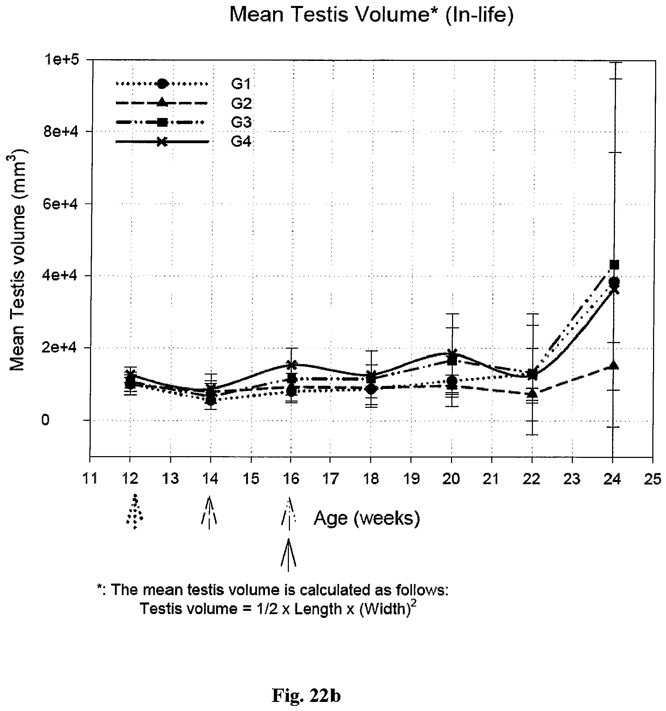

**Figures 22a and 22b** illustrate the testis length (Fig. 22a) and volume (Fig. 22b) after immunization of male pigs with formulations containing LHRH1 (SEQ ID NO: 10) in adjuvant ISA50V2. There was no difference in the weights of testis pair, epididymis pair and the testis lengths amongst pigs in Groups 1 to 4. The arrows indicate the boost time point according to the respective groups.

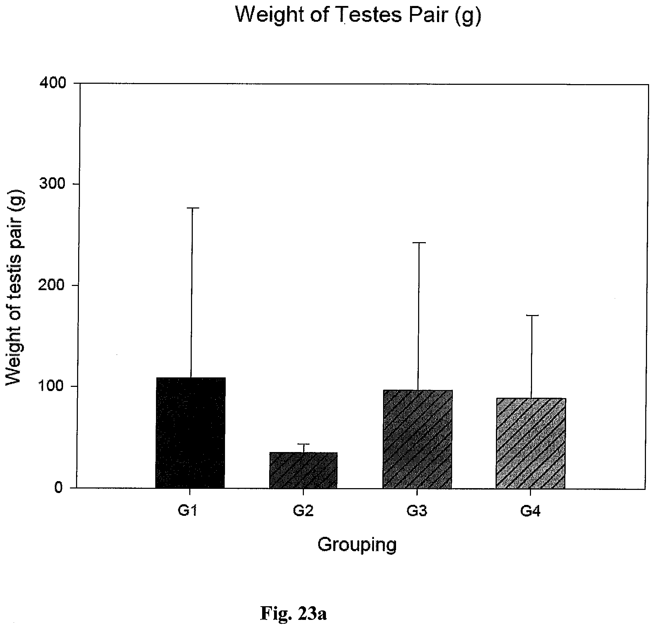

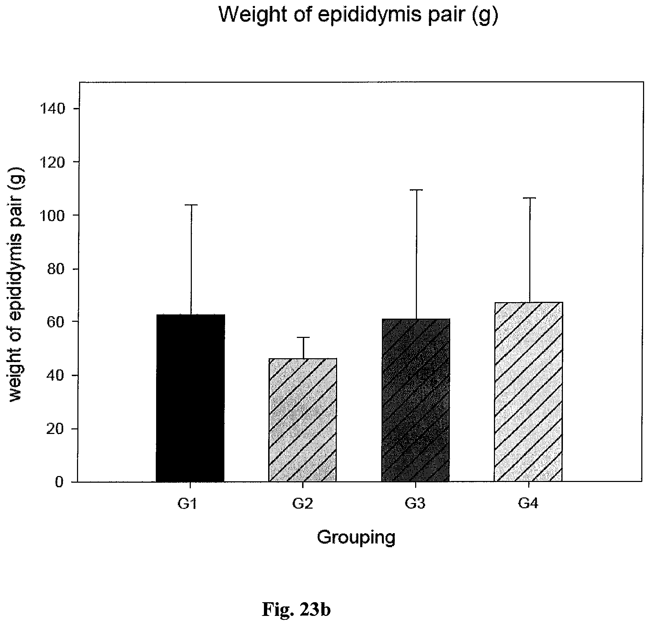

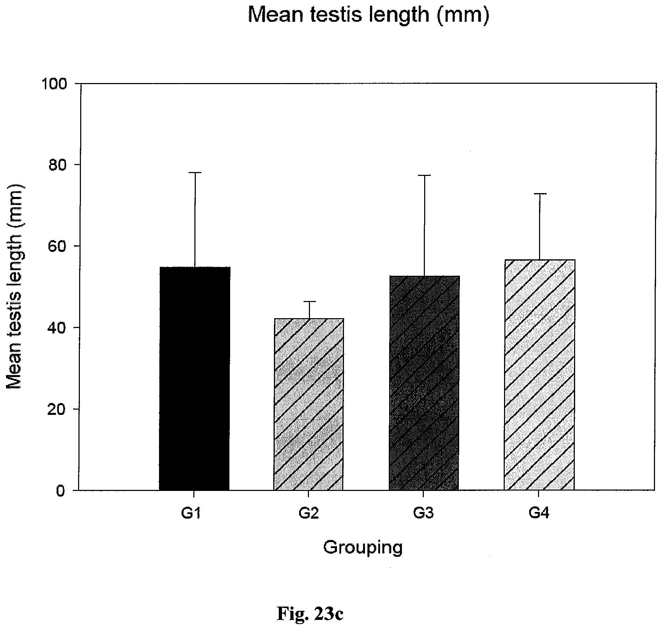

**Figures 23a, 23b, and 23c** illustrate the weights of testis pair (Fig. 23a), epididymis pair (Fig. 23b) and the testis lengths (Fig. 23c) in pigs at necropsy after immunization of male pigs with formulations containing LHRH1 (SEQ ID NO: 10) in adjuvant ISA50V2.

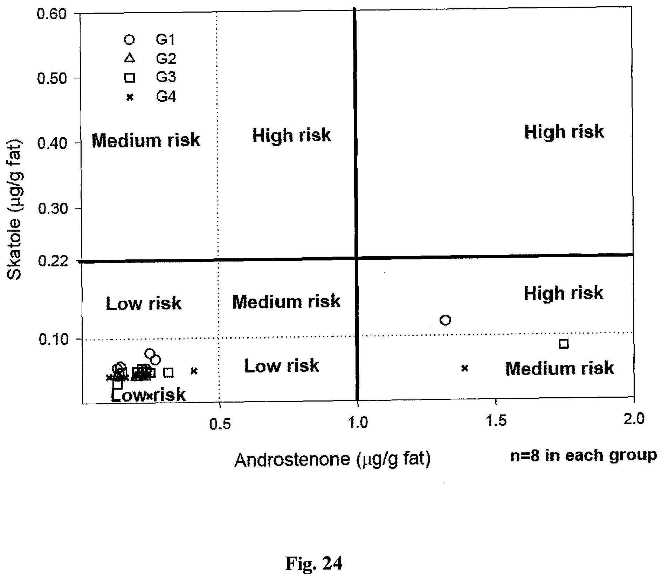

**Figure 24** illustrates the androstenone and skatole concentrations at 24 weeks of age after immunization of male pigs with formulations containing LHRH1 (SEQ ID NO: 10) in adjuvant ISA50V2. In the boar taint factor distribution plot, the risk factors for all pigs in group 1-4 were located in the low/medium risk except for one pig in group 1.

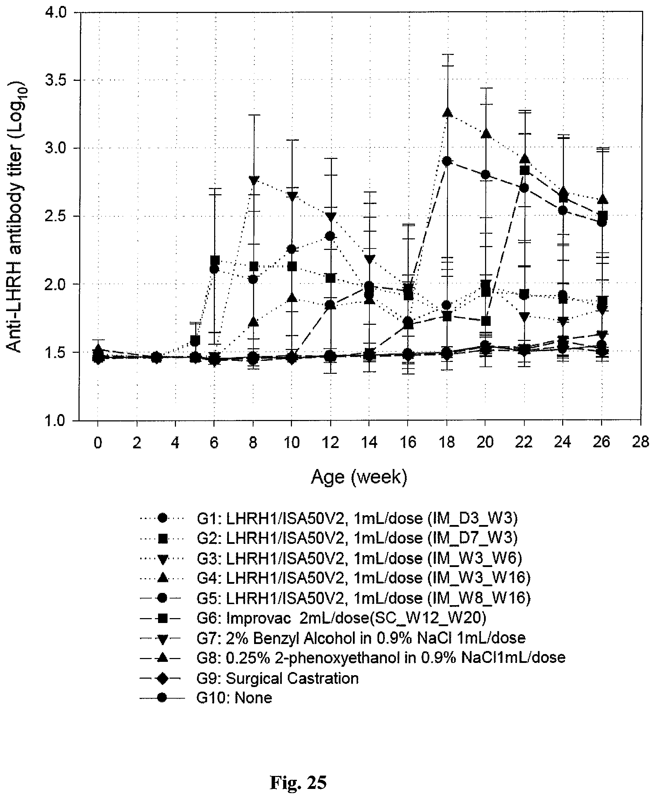

**Figure 25** illustrates the anti-LHRH antibody titers after immunization of male pigs with formulations containing LHRH1 (SEQ ID NO: 10) in adjuvant ISA50V2. The titers of anti-LHRH antibodies at 0 and 3 weeks of age were around the background level at 1.718 ± 0.297, 1.765 ± 0.340 and 1.770 ± 0.283 (Log10) for pigs in Groups 1, 2, and 3, respectively. The anti-LHRH antibody titers were 3.249 ± 0.346 in Group 4 and 2.893 ± 0.786 in Group 5 at 18 weeks of age, after boost at the age of 16 weeks.

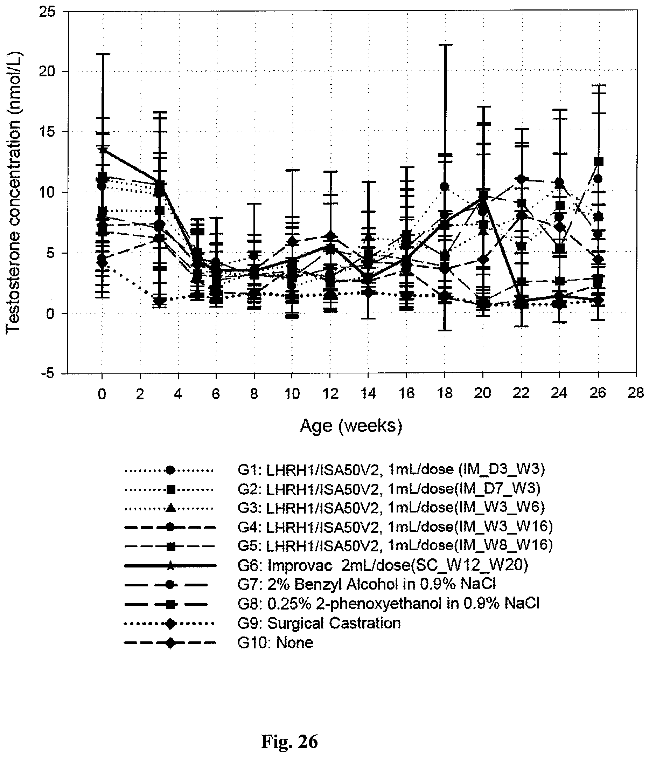

**Figure 26** illustrates the serum testosterone concentrations in pigs after immunization with formulations containing LHRH1 (SEQ ID NO: 10) in adjuvant ISA50V2. The serum testosterone levels of pigs in Groups 1 and 2 were 2.154 ± 0.921 nmol/L at 10 weeks of age and 2.183 ± 1.231 nmol/L at 6 weeks of age, respectively. The lowest testosterone level in pigs of Group 3 was 3.065 ± 0.205 nmol/L at 12 weeks of age. The serum testosterone concentrations were suppressed to low levels at around 0.586 ± 0.184 and 0.893 ± 1.192 nmol/L for Groups 4 and 5 at 20 weeks of age, respectively. In comparison, the serum testosterone level in pigs receiving vaccine Improvac® (Group 6) remained at a high concentration (about 9.415 ± 7.560 nmol/L) at 20 weeks of age and then decreased to a low concentration at 22 weeks of age.

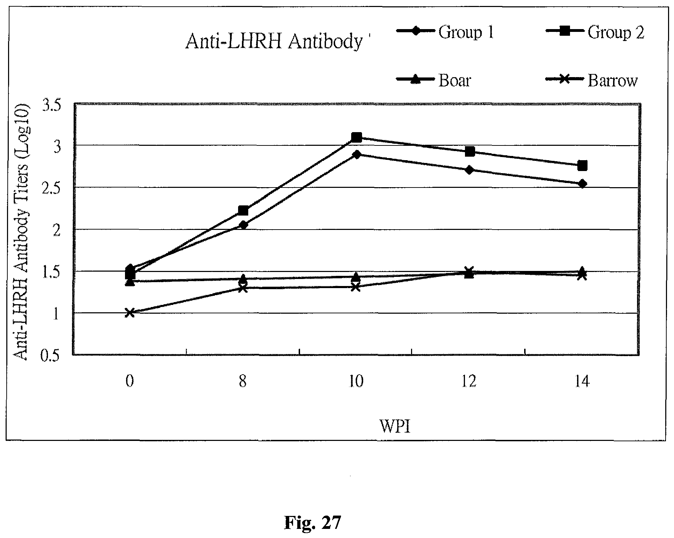

**Figure 27** illustrates the anti-LHRH antibody titers after immunization of male pigs with formulations containing LHRH3 (SEQ ID NO: 7, 8, and 9) in adjuvant ISA50V. The anti-LHRH antibody titers in pigs of Groups 1 and 2 were significantly higher than those in Groups 3 (intact unimmunized) and 4 (surgically castrated) controls.

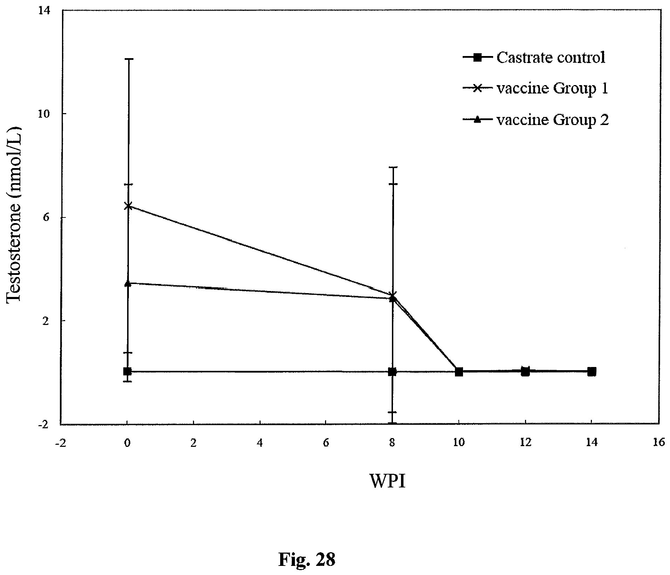

**Figure 28** illustrates complete (100%) immunocastration of male pigs in Groups 1 and 2 after immunization with formulation containing LHRH3 (SEQ ID NO: 7, 8, and 9) in adjuvant ISA50V at different dosages.

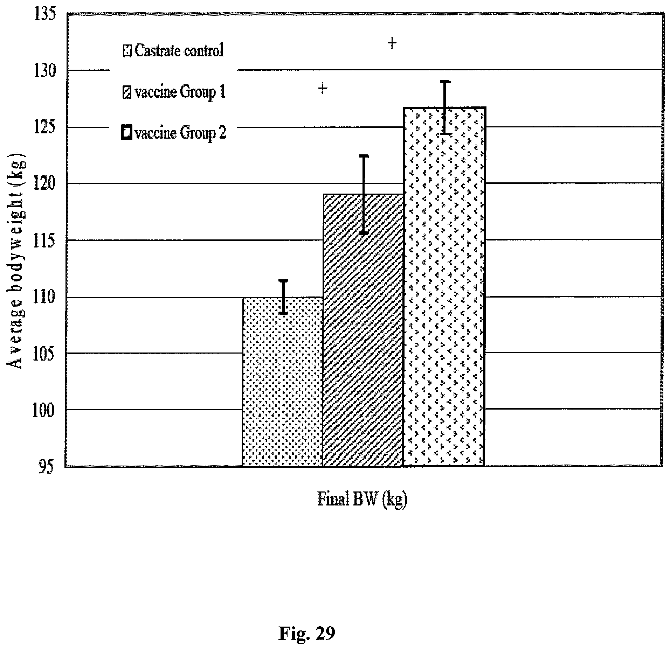

**Figure 29** illustrates higher Average Daily Gain (ADG) due to increased Average Daily Feed Intake (ADFI) for male pigs receiving immunization with formulation containing LHRH3 (SEQ ID NO: 7, 8, and 9) in adjuvant ISA50V when compared to the surgical castration group.

## DETAILED DESCRIPTION OF THE INVENTION

The following description is of the best-contemplated mode to carry out the invention. This description is for purpose of illustrating the general principles of the invention and should not be taken in a limiting sense. The scope of the invention is best determined by reference to the appended claims.

The present invention provides peptides having LHRH, a 10-mer peptide linked through its N-terminus to a co-stimulatory or helper T cell epitope (Th epitope).

The term "LHRH" refers to Luteinizing-Hormone-Releasing Hormone (GenBank: AAB34379.1), which is a trophic peptide hormone responsible for the release of follicle-stimulating hormone (FSH) and luteinizing hormone (LH) from the anterior pituitary. The term LHRH used in this disclosure includes homologues of LHRH derived from different phyla including birds, fish, reptiles and invertebrates. The peptides of LHRH preferably comprise an amino acid sequence of SEQ ID NO: 1 as shown in Table 1.

The term "vaccine" is a general clinical term to describe an immunostimulatory composition that leads to the production of antibodies in a subject that has been exposed to the vaccine. From a clinical perspective, subjects that have been administered vaccine compositions will generate antibodies to the antigen present in the vaccine. Vaccines compositions described in the present application are not limited to biological preparations that improve immunity against a particular disease or pathogen.

The term "helper T cell epitope (Th epitope)" of the invention refers to Th epitopes derived from foreign pathogens including but not limited to, as examples, hepatitis B surface (HBsAg) and core antigen (HBc) helper T cell epitopes, pertussis toxin helper T cell epitopes (PT Th), tetanus toxin helper T cell epitopes (TT Th), measles virus F protein helper T cell epitopes (MVF Th), Chlamydia trachomatis major outer membrane protein helper T cell epitopes (CT Th), diphtheria toxin helper T cell epitopes (DT Th), Plasmodium falciparum circumsporozoite helper T cell epitopes (PF Th), Schistosoma mansoni triose phosphate isomerase helper T cell epitopes (SM Th), Escherichia coli TraT helper T cell epitopes (TraT Th). The pathogen-derived Th selected here as representative examples of promiscuous Th were listed as SEQ ID NOs: 2-9 and 42-52 in U.S. Pat. No. 5,759,551 and are incorporated herein by reference.

Useful Th epitopes may also include combinatorial Th epitopes. In Wang et al. (WO 95/11998), a particular class of combinatorial Th epitopes, a "Structured Synthetic Antigen Library" (SSAL) was described. Th SSAL epitopes comprise a multitude of Th epitopes with amino acid sequences organized around a structural framework of invariant residues with substitutions at specific positions. The sequences of the SSAL are determined by retaining relatively invariant residues while varying other residues to provide recognition of the diverse MHC restriction elements. This may be accomplished by aligning the primary amino acid sequence of a promiscuous Th, selecting and retaining as the skeletal framework, the residues responsible for the unique structure of the Th peptide, and varying the remaining residues in accordance with known MHC restriction elements. Lists of the invariant and variable positions with the preferred amino acids of MHC restriction elements are available to obtain MHC-binding motifs. These may be consulted in designing SSAL Th epitopes (Meister et al., Vaccine, 1995; 13:581-591). In one embodiment, the Th epitope includes SEQ ID NOs: 2, 3, 4, and/or 5 as shown in Table 1.

SEQ ID No: 6, refers to a peptide fragment derived from the invasin protein of the pathogenic bacteria Yersinia spp., a microbial outer membrane protein which mediates entry of the bacteria into mammalian cells Invasin of cultured mammalian cells by the bacterium was demonstrated to require interaction between the Yersinia invasin molecule and several species of the $\beta1$ family of integrins present on the cultured cells. Since T lymphocytes are rich in $\beta1$ integrins (especially activated immune or memory T cells), and the demonstrated T cell co-stimulatory properties associated with this invasin domain, it can be linked to promiscuous Th epitope comprising LHRH constructs to further enhance the immunogenicity of a designed peptide immunogen (U.S. Pat. No. 6,025,468).

The peptides of the invention include at least one LHRH, a Th epitope and optionally a co-stimulatory invasin peptide domain. The LHRH peptide (including homologues thereof) can be covalently linked through its N-terminal amino acids with a spacer to a peptide containing at least one sequence known to contain a Th epitope. The Th epitope can be covalently linked through its N-terminus to the co-stimulatory invasion peptide domain. The spacer includes, but is not limited to, Gly-Gly, Lys, $\epsilon$NLys, $\epsilon$NLys-(Lys)n where n=1 to 3, or Lys-Lys-Lys-$\epsilon$NLys (SEQ ID NO: 11), etc.

The peptides of the invention have from about 20 to about 100 amino acid residues, preferably from about 20 to about 70 amino acid residues and more preferably from about 25 to about 50 amino acid residues. In another preferred embodiment, the peptide has from about 27 to about 45 amino acid residues.

The number of Th epitope includes, but are not limited to, one, two, three, four, or more of the Th peptides as shown in Table 1 the invention. The peptides of the invention comprise at least one peptide of Th epitopes selected from SEQ ID NOs: 2, 3, 4, 5 which can be optionally linked to the N-terminus of the LHRH peptide of SEQ ID NO: 1. In one embodiment, Th epitopes may be linked to the N-terminus of LHRH to form a peptide of the invention. For example, the peptides of SEQ ID NOs: 2 and 3 may be sequentially linked to the N-terminus of LHRH by spacers (Gly-Gly, Lys, or $\epsilon$NLys). One skilled in the art would change the number of the Th epitope and kinds of spacers linked to LHRH peptide immunogen to obtain an optimal peptide of the invention, if necessary.

In another embodiment, the peptides of the invention comprise SEQ ID NOs: 7, 8, 9, and/or 10 as shown in Table 2, or having at least 60%, at least 65%, at least 70%, at least 75%, at least 80%, at least 85%, at least 90%, at least 95%, or at least 99% similarity thereto.

The peptides of this invention can be made by synthetic chemical methods which are well known to the ordinarily skilled artisan; See, for example, Grant, ed. (1992) Synthetic Peptides: A User's Guide, W.H. Freeman & Co., New York, N.Y, pp. 382. Hence, peptides can be synthesized using the automated solid phase synthesis with either t-butyloxycarbonyl (t-Boc) or 9-fluorenyl-methyloxycarbonyl (F-moc) chemistry on an Applied Biosystems Peptide Synthesizer Model 430A or 431. To synthesize a poly lysine core moiety, unprotected Di(t-Boc) or Di(F-moc)-N$\alpha$, N$\epsilon$) lysine residues are used in place of t-Boc or F-moc with a protected $\epsilon$-amino group. To improve the solubility of a designed peptide, the peptide can be elongated with additional serine or/and lysine residues at the N-terminus. After complete assembly of the desired peptide, the resin is treated according to standard procedures to cleave the peptide from the resin and deblock the protecting groups on the amino acid side chains. The free peptide is purified by HPLC and characterized biochemically. Alternatively, the longer linear peptides can be synthesized by well known recombinant DNA techniques. Any standard manual on DNA technology provides detailed protocols to produce the peptides of the invention.

The peptide of the invention when used as the key ingredient in a vaccine formulation can yield immunocastration including loss of the physical and/or chemical characteristics associated with sexual maturity such as sexuality, fighting, wandering, aggressive sexual behavior, unwanted organoleptic characteristics, tumors of reproductive organs and pregnancy, oestrous cycling, fertility, pregnancy, and tumors of the reproductive organs in an immunized animal. Accordingly, inhibiting the characteristics includes inhibiting sexual activity (for example, preventing male cattle mounting other male cattle), preventing or delaying ovulation, shrinking the testes and epididymides, reducing testosterone concentration, reducing aggressive behavior or reducing unwanted organoleptic characteristics such as boar taint.

The peptides of the invention are formulated for convenient and effective administration in effective amounts with a suitable pharmaceutically acceptable carrier in dosage unit form as herein disclosed. A unit dosage form can, for example, contain the principal peptide antigen in amounts ranging from 0.5 $\mu$g to about 2,000 $\mu$g, generally, preferably, more than 6.25$\mu$g, most preferably, 25 $\mu$g, 30 $\mu$g, 40 $\mu$g, 50 $\mu$g, 60 $\mu$g, 70 $\mu$g, 80 $\mu$g, 90 $\mu$g, 100, 150 or 200 $\mu$g. In the case of compositions containing supplementary active ingredients, the dosages are determined by reference to the usual dose and manner of administration of the said ingredients.

This invention also provides compositions comprising pharmaceutically acceptable delivery systems for the administration of the peptide immunogens. The compositions comprise an immunologically effective amount of at least one peptide of this invention. The number of the peptide of the invention includes, but is not limited to, one, two, three, four, or more in a composition (vaccine) of the invention. For example, the composition of the invention comprises SEQ ID No: 7, 8, 9, 10, or a mixture thereof. In one embodiment, the composition of the invention includes a single peptide of SEQ ID No: 7, 8, 9, or 10. In another embodiment, the composition of the invention includes SEQ ID Nos: 7, 8, and 9 which is referred to LHRH3 in the following examples. In another embodiment, the composition of the invention includes SEQ ID No: 10 which is referred to LHRH1 in the following examples. When so formulated, the compositions of the present invention comprising LHRH or a homologue thereof as target antigenic site, are used for prevention/removal of boar taint, enhanced growth profile, immunocastration of pigs, and for contraception in males and females.

The peptide immunogens of the invention can be formulated as immunogenic compositions using adjuvants, emulsifiers, pharmaceutically-acceptable carriers or other ingredients routinely provided in vaccine compositions.

Adjuvants or emulsifiers which can be used in this invention include alum, incomplete Freund's adjuvant (IFA), liposyn, saponin, squalene, L121, emulsigen, monophosphoryl lipid A (MPL), QS21, and ISA 720, ISA 50, ISA 50V2, ISA 35 or ISA 206 as well as the other efficacious adjuvants and emulsifiers. The formulations are readily determined by one of ordinary skill in the art and also include formulations for immediate release and/or for sustained release. The present vaccines can be administered by any convenient route including subcutaneous, oral, intramuscular, intraperitoneal, or other parenteral or enteral route. Similarly the immunogens can be administered in a single dose or multiple doses. Immunization schedules are readily determined by the ordinarily skilled artisan.

In a particular embodiment, the delivery vehicle and adjuvant is Montanide™ ISA 50V2 (an oil vaccine adjuvant composition comprised of vegetable oil and mannide oleate for production of water-in oil (i.e. w/o) emulsions), Tween® 80 (also known as: Polysorbate 80 or Polyoxyethylene (20) sorbitan monooleate), a CpG oligonucleotide, and/or any combination thereof. In another embodiment, the pharmaceutical composition is a water-in-oil-in-water (i.e. w/o/w) emulsion with Emulsigen or Emulsigen D as the adjuvant. Also provided are other ingredients routinely incorporated with vaccine formulations, and instructions for dosage such that a balanced B and T cell immune response is generated.

The pharmaceutical forms suitable for injectable use include sterile aqueous solutions (where water soluble) or dispersions and sterile powders for the extemporaneous preparation of sterile injectable solutions or dispersion. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption or delayed release of the injectable compositions can be brought about by the use in the compositions of agents delaying absorption, for example, aluminum monostearate or/and gelatin.

The invention also provides a method for inducing the titer of anti-LHRH antibodies, decrease of testosterone concentration, prevention of boar taint, shrinkage of testes and epididymides, immunocastration of pigs, and for contraception by administering the subject peptide compositions to the mammals for a time.

Generally, the method of the invention can induce the titer of anti-LHRH antibodies in a pig, and the titer of anti-LHRH antibodies is more than 2.0, preferably, 2.5, or 3.0 (Logio) after one or two vaccinations. The method of the invention also can suppress the testosterone concentration in pigs, and the testosterone concentration is less than 2.5 nmol/L, preferably, 2.0, 1.87, or 1.0 nmol/L after one or two vaccinations. Further, the method of the invention can increase the body weight of pigs, preferably with an increase by 10% or more.

The compositions of the invention may be administered by any suitable method known in the art, including, but not limited to, oral administration or by injection, preferably, intramuscular injection.

The composition of the instant invention contains an effective amount of one or more of the peptide immunogens of the present invention and a pharmaceutically acceptable carrier. Such a composition in a suitable dosage unit form generally contains about 0.5 $\mu$g to about 1 mg of the peptide immunogen per kg body weight. When delivered in multiple doses, it may be conveniently divided into an appropriate amount per dose. For example, the dose, e.g. 6.25 $\mu$g to 200 $\mu$g; preferably 50 $\mu$g, may be administered by injection, preferably intramuscularly. This may be followed by repeat (booster) doses. Dosage will depend on the age, weight and general health of the subject as is well known in the vaccine and therapeutic arts.

The invention also provides a method for inhibiting characteristics induced by the sexual maturation of pigs, comprising administering an effective amount of a vaccine composition of the invention.

The number and timing of doses are not limited in the method of the invention. Generally, the method of the invention includes at least one dose, preferably, two, three, four, five doses or more, preferably, two doses or more. One skilled in the art would easily know to increase the number of does to induce the efficiency. The first dose can be delivered to pigs at any age (e.g., 1, 2, 3, 4, 5, 6, 7, 8, 9, 10 weeks old). The time points of vaccination can be changed or modified depending on different types of pigs, dosage, adjuvant, and administration method.

Generally, prime vaccination (first dose) is done after 3, preferably 3 to 8, weeks of age. A boost vaccination (second dose) is preferably given between 3 and 13 weeks after the priming. The standard scheme would have 3 to 13 weeks between priming and boosting.

In one embodiment, a preferred vaccination schedule would be the following: the priming would be given about 8 weeks of age, the booster would be given about 16 weeks of age, and in cases where pigs are slaughtered after 6 to 8 weeks of booster.

The prime/boost vaccination schedule of the invention is suitable for pigs. Depending on the production methods used in different countries, in many cases pigs may not reach the age of 21 weeks.

Specific embodiments of the present invention include, but are not limited to, the following:

(1) A vaccine composition for castrating pigs, comprising a peptide immunogen and a veterinarily acceptable delivery vehicle or adjuvant, wherein the peptide immunogen comprises

(a) a LHRH peptide of SEQ ID NO: 1, and
(b) at least one T helper epitope selected from a group consisting of SEQ ID NOs: 2, 3, 4, and 5, and, optionally, an immunostimulatory peptide of SEQ IN NO:6, wherein the LHRH peptide is covalently linked through its N-terminus residue to the T helper epitope.

(2) The vaccine composition according to (1), wherein the peptide of LHRH, T helper epitope, and, optionally, an immunostimulatory peptide, is linked by Gly-Gly, $\varepsilon$NLys, Lys-$\varepsilon$NLys, Lys-Lys-$\varepsilon$NLys, Lys-Lys-Lys-$\varepsilon$NLys (SEQ ID NO: 11), $\varepsilon$NLys-Lys-Lys, or $\varepsilon$NLys-Lys-Lys-Lys (SEQ ID NO: 12).
(3) The vaccine composition according to (1), wherein the peptide immunogen comprises SEQ ID NOs: 7, 8, 9, and/or 10, or a mixture thereof.
(4) The vaccine composition according to (1), wherein the veterinarily acceptable adjuvant comprises ISA50, ISA50V2, or Emulsigen D.
(5) The vaccine composition according to (1), wherein the total amount of the peptide immunogen is about 12.5 $\mu$g to 200 $\mu$g per dose.
(6) A method for inhibiting characteristics induced by the sexual maturation of a pig, comprising administering an effective amount of a vaccine composition of claim 1 to a pig.
(7) The method according to (6), wherein the characteristic comprises boar taint, sexual activity, sexuality, fertility, and estrous behavior.
(8) The method according to (6), wherein the vaccine composition is administered by intramuscular or subcutaneous injection.
(9) The method according to (6), wherein first dose of the vaccine composition is applied to the pig at the age of 3 to 8 weeks old.
(10) The method according to (6), wherein the second dose of the vaccine composition is applied to the pig at the age of 6 to 16 weeks old.

Additional specific embodiments of the present invention include, but are not limited to the following:

(1) A veterinary composition comprising:

(a) a peptide immunogen selected from the group consisting of

(i) a mixture of SEQ ID NOs: 7, 8, and 9,
(ii) SEQ ID NO: 10, and
(iii) a combination of (i) and (ii); and

(b) a veterinarily acceptable delivery vehicle or adjuvant.

(2) The veterinary composition according to (1), wherein the peptide immunogen in (a) is (i) a mixture of SEQ ID NOs: 7, 8, and 9.
(3) The composition according to (2), wherein the veterinarily acceptable adjuvant comprises ISA50, ISA50V2 or Emulsigen D.
(4) The composition according to (2), wherein the total amount of the peptide immunogen is about 12.5 μg to 200 μg per dose.
(5) A method for inhibiting characteristics induced by the sexual maturation of a pig, comprising administering an effective amount of the composition of (2) to a pig.
(6) The method according to (5), wherein the characteristics comprise boar taint, sexual activity, sexuality, fertility, and estrous behavior.
(7) The method according to (5), wherein the composition is administered by intramuscular or subcutaneous injection.
(8) The method according to (5), wherein first dose of the composition is applied to the pig at the age of 3 to 8 weeks old.
(9) The method according to (5), wherein the second dose of the composition is applied to the pig at the age of 6 to 16 weeks old.
(10) A method for reducing the production of testosterone and its derivatives in an animal comprising administering an effective amount of the composition of (2) to the animal.
(11) The veterinary composition according to (1), wherein the peptide immunogen in (a) is (ii) SEQ ID NO: 10.
(12) The composition according to (11), wherein the veterinarily acceptable adjuvant comprises ISA50, ISA50V2 or Emulsigen D.
(13) The composition according to (11), wherein the total amount of the peptide immunogen is about 12.5 μg to 200 μg per dose.
(14) A method for inhibiting characteristics induced by the sexual maturation of a pig, comprising administering an effective amount of the composition of (11) to a pig.
(15) The method according to (14), wherein the characteristics comprise boar taint, sexual activity, sexuality, fertility, and estrous behavior.
(16) The method according to (14), wherein the composition is administered by intramuscular or subcutaneous injection.
(17) The method according to (14), wherein first dose of the composition is applied to the pig at the age of 3 to 8 weeks old.
(18) The method according to (14), wherein the second dose of the composition is applied to the pig at the age of 6 to 16 weeks old.
(19) A method for reducing the production of testosterone and its derivatives in an animal comprising administering an effective amount of the composition of (11) to the animal.

## EXAMPLE 1

### Synthesis of LHRH Peptide

Methods for synthesizing LHRH related peptide constructs that were included in the development effort for an efficacious targeting LHRH vaccine design and formulation are described below. The peptides can be synthesized in small-scale amounts, which are useful for laboratory pilot and field studies, as well as large-scale (kilogram) amounts, which are useful for industrial/commercial production of vaccine formulations and serological assays.

A large repertoire of LHRH related antigenic peptides having sequences with lengths from approximately 10 to 40 amino acids were designed for the screening and selection of the most optimal peptide constructs for use in an efficacious LHRH vaccine. Each construct contains an LHRH peptide (SEQ ID NO: 1) synthetically links to a carefully designed helper T cell (Th) epitope or an immunostimulatory peptide, identified in Table 1 (SEQ ID NOs: 2 to 6). The LHRH peptides used in the LHRH vaccine of the invention are SEQ ID NOs: 7 to 10.

All peptides used for immunogenicity studies or related serological tests for detection and/or measurement of anti-LHRH antibodies were synthesized on a small scale using Fmoc chemistry by peptide synthesizers of Applied BioSystems Models 430A, 431 and/or 433. Each peptide was produced by an independent synthesis on a solid-phase support, with Fmoc protection at the N-terminus and side chain protecting groups of trifunctional amino acids. Completed peptides were cleaved from the solid support and side chain protecting groups were removed by 90% Trifluoroacetic acid (TFA). Synthetic peptide preparations were evaluated by Matrix-Assisted Laser Desorption/Ionization-Time-Of-Flight (MALDI-TOF) Mass Spectrometry to ensure correct amino acid content. Each synthetic peptide was also evaluated by Reverse Phase HPLC (RP-HPLC) to confirm the synthesis profile and concentration of the preparation.

Despite rigorous control of the synthesis process (including stepwise monitoring the coupling efficiency), peptide analogues were also produced due to unintended events during elongation cycles, including amino acid insertion, deletion, substitution, and premature termination. Thus, synthesized preparations typically included multiple peptide analogues along with the targeted peptide. Despite the inclusion of such unintended peptide analogues, the resulting synthesized peptide preparations were nevertheless suitable for use in immunological applications including immunodiagnosis (as antibody capture antigens) and vaccination (as peptide immunogens). Typically, such peptide analogues, either intentionally designed or generated through synthetic process as a mixture of byproducts, are frequently as effective as a purified preparation of the desired peptide, as long as a discerning QC procedure is developed to monitor both the manufacturing process and the product evaluation process to guarantee the reproducibility and efficacy of the final product employing these peptides. Large scale peptide syntheses in the multi-hundred to kilo gram quantities were conducted on a customized automated peptide synthesizer UBI 2003 at 15 mmole to 50 mmole scale according to the same solid phase peptide synthesis principle.

For active ingredients used in the final vaccine formulations for field trials, LHRH peptide constructs were purified by preparative RP-HPLC under a shallow elution gradient and characterized by MALDI-TOF mass spectrometry, amino acid analysis and RP-HPLC for purity and identity.

## EXAMPLE 2

### Preparation of the Vaccine Formulation

A mixture of three LHRH peptide immunogens (LHRH3: SEQ ID NOs: 7, 8 and 9) and a single peptide immunogen (LHRH1: SEQ ID NO: 10) were formulated respectively in an water in oil (W/O) emulsion delivery system using an ISA adjuvant from Seppic (France) or in a water in oil in water (W/O/W) Emulsigen D from MVP (USA). Briefly, peptides in saline solution (20% w/v NaCl solution) were combined in equal molar ratios, filtered aseptically (with 0.22 micron filter) and then mixed with delivery vehicle ISA50V2 or Emulsigen D adjuvant through homogenization. The formulation processes were monitored throughout for viscosity. Final products were characterized by identity test, physical test, and sterility test. All formulation, filling and packaging procedures were performed in a clean room to maintain the sterile condition.

## EXAMPLE 3

### Immunization of Male Pigs with Varying Doses of LHRH3 (SEQ ID NOs: 7, 8, and 9) Vaccine Formulations

A total of 40 boars at 8 weeks of age and 8 surgical castrated pigs were used for the study. These pigs were divided into groups with 8 pigs per group as shown in Tables 3 and 4 with the LHRH3 (SEQ ID NOs: 7, 8, and 9) formulated with oil based adjuvant (MontanideTM ISA50V or ISA50V2) to form an water-in-oil (W/O) emulsion as shown in Table 3 (Groups 1 to 4), or with oil based adjuvant Emulsigen D to form the water in oil in water (W/O/W) emulsion as shown in Table 4 (Groups 8 to 11), to enhance the immunogenicity of the finished vaccine products. Three control groups included pigs receiving saline as the negative controls (Groups 5 and 12), pigs having been surgically castrated as the positive controls (Groups 6 and 13), and pigs receiving the state-of-the-art LHRH vaccine Improvac® for direct efficacy comparison (Groups 7 and 14).

Blood samples were collected at 0, 4, 8, 10, 12, 14, and 16 weeks post initial immunization (or WPI) for measurement of the serum titers of anti-LHRH antibodies and the serum concentrations of testosterone, respectively in pigs.

### a. Titration of Serum anti-LHRH antibodies by Enzyme-Linked Immunosorbent Assay (ELISA).

ELISA assay used for evaluating serum titers of anti-LHRH antibodies were developed and described below.

The wells of 96-well plates were coated individually for 1 hour at 37°C with 100 μL of individual target peptides, at 2 μg/mL individually or as an equal molar mixture, unless specifically mentioned, in 10 mM $NaHCO_3$ buffer, pH 9.5 unless noted otherwise.

The peptide-coated wells were incubated with 250 μL of 3% by weight of gelatin in PBS in 37°C for 1 hour to block non-specific protein binding sites, followed by three washes with PBS containing 0.05% by volume of Tween® 20 and dried. 100 μL of the diluted sera samples were added to each of the wells and allowed to react for 60 minutes at 37°C. The wells were then washed six times with 0.05% by volume Tween® 20 in PBS to remove unbound antibodies.

100 μL of the peroxidase-labeled goat anti-swine IgG at a pretitered optimal dilution and in 1% by volume normal goat serum with 0.05% by volume Tween® 20 in PBS, was added to each well and incubated at 37°C for another 30 minutes. The wells were washed six times with 0.05% by volume Tween® 20 in PBS to remove unbound antibody and reacted with 100 μL of the substrate mixture containing 0.04% by weight 3', 3', 5', 5'-Tetramethylbenzidine (TMB) and 0.12% by volume hydrogen peroxide in sodium citrate buffer for another 15 minutes. This substrate mixture was used to detect the peroxidase label by forming a colored product. Reactions were stopped by the addition of 100 μL of 1.0 M $H_2SO_4$ and absorbance at 450 nm ($A_{450}$) determined.

Serum dilutions were carried out in accordance with the purpose for measuring serum titers of LHRH antibodies. For the determination of antibody titers in pigs that received peptide-based LHRH vaccine formulations, a 10-fold serial dilution of sera from 1:10 to 1:10,000 was performed on the ELISA and the titer of a tested serum, expressed as $Log_{10}$, was calculated by linear regression analysis of the $A_{450}$.

According to **Figs. 1a** and **1b,** except for the negative (Groups 5 and 12) and surgical castration (Groups 6 and 13) groups, all groups containing SEQ ID NOs: 7, 8, and 9 tested demonstrated a high titer of LHRH antibodies after two weeks of boost at 10 WPI (18 weeks old). The antibody titers of the vaccinated groups (Groups 1 to 4 and Groups 8 to 11) were maintained near or above 3.0 ($Log_{10}$) until 16 WPI (24 weeks of age), a time for sale of pigs on the market, for not only the groups formulated in ISA50V2 (Groups 1 to 4) but also the groups formulated in Emulsigen D (Groups 8 to 11). In contrast, the antibody titers of pigs receiving Improvac® (Group 7) only rose at 14 WPI, 2 weeks after the boost, with antibody titers lower than that of the peptide vaccine of the invention (Groups 1 to 4 and Groups 8 to 11).

## b. Assessment of Serum Testosterone Concentration

Serum Testosterone (TT) concentration was measured by commercial ELISA or RIA kits. The kits included the DRG® Testosterone ELISA Kit (EIA-5179), BioVendor ELISA kit, and the Seamans RIA kit. Testosterone concentrations in serum of Groups 1 to 4 and Groups 8 to 11 formulated in ISA50V2 or Emulsigen D respectively were measured in this particular example by DRG® Testosterone ELISA Kit which correlated reversely to the corresponding serum antibody titers in pigs with the concentrations falling below the castration cut-off value (1.87 nmol/L in general or 1.0 nmol/L in more strict criteria) two weeks after the boost (i.e. 10 WPI or pigs at 18 weeks of age). Pigs in all other groups formulated in ISA50V2 or Emulsigen D maintained a castration level of testosterone, as shown in **Figs. 2a** and **2b,** up to 16 WPI (i.e. 24 weeks of age), the window for sale of pigs on the market. The results showed that LHRH3 vaccine formulation of the invention displayed a dose-dependent inhibition of testosterone, and Groups 4 and 11 provided a higher inhibition level compared to other groups.

Compared to pigs receiving the vaccine formulations, pigs in the negative and castration control groups always maintained a background level of low anti-LHRH antibody titers with the serum testosterone concentrations fluctuating in the negative controls (Groups 5 and 12) as the uncastrated animals; for pigs in the surgically castrated positive controls (Groups 6 and 13), the serum testosterone concentrations all remained at the low castrating level.

Those pigs receiving LHRH vaccine Improvac® maintained a high level of serum testosterone concentration until 12 WPI when the boost was given and such level was decreased after 14 WPI, i.e. two weeks after the boost. The results showed clearly that Improvac® is not as potent as the vaccine formulations of the invention (Groups 1 to 4 and Groups 8 to 11, containing peptide SEQ ID NOs: 7, 8, and 9 formulated in ISA50V2 or Emulsigen D) for immunocastration of pigs.

## c. Body Weight

According to **Figs 3a** and **3b,** the pig body weight was monitored at all times from pre-immunization (3 weeks of age) to the end of the study (16 WPI, 24 weeks of age). The results revealed the influence of the vaccination over the animal's body weight. The pigs receiving saline as the negative controls and those received surgical castration as the positive controls all had significantly lower average body weights of 94.9 kg (negative control, groups 5 and 12) and 88.8 kg (surgical castration control, Groups 6 and 13) respectively at 24 weeks of age. In comparison to pigs receiving surgical castration (Groups 6 and 13), pigs receiving the vaccine formulations of the invention at 200ug/mL/dose (Groups 4 and 11) had a significantly greater economic benefit in body weight gain by about 27.9%.

## EXAMPLE 4

**Immunization of Male Pigs with Varying Doses of LHRH1 (SEQ ID NO: 10) Vaccine Formulations**

In this Example, the protocol was similar to the protocol of Example 3, except that the peptide immunogen in the current vaccine formulation was changed to SEQ ID NO: 10, and the dosage was changed according to Tables 5 and 6. After immunization, sera from all animals were collected at multiple time points for determination of the titers of LHRH antibodies and the corresponding concentrations of serum testosterone. Additionally, the sexual organs (testes and epididymides) of pigs at 24 weeks of age were also collected for weight measurement at the time of sacrifice.

### a. Titration of Serum Anti-LHRH Antibodies by ELISA

According to **Figs. 4a** and **4b,** except for the negative (saline) and positive (surgical castration) control groups, all of the testing groups containing peptide immunogen SEQ ID NO: 10 had a high titer of antibody against LHRH after two weeks of boost at 10 WPI (18 weeks of age). The antibody titers were maintained near or above 3.0 (Log10) until 16 WPI (24 weeks of age), the window for sale of pigs on the market, for not only the pigs in Groups 1 to 4 formulated in adjuvant ISA50V2 but also the pigs in Groups 7 to 10 formulated in adjuvant Emulsigen D.

### b. Assessment of Serum Testosterone Concentration

According to **Figs. 5a** and **5b,** serum testosterone concentrations of pigs in Groups 1-4 formulated in ISA50V2 or in Groups 7-10 formulated in Emulsigen D all correlated reversely with the corresponding antibody titers and all achieved a level below the castration cut-off value (<1.87 nmol/L) two weeks after the boost (i.e. 10 WPI, 18 weeks of age). Except for the lowest dosage in Groups 1 and 7, pigs in all other groups formulated in ISA50V2 or Emulsigen D maintained a castration level of serum testosterone until 16 WPI (24 weeks of age) which is the window for sale of pigs on the market.

### c. Body Weight

According to **Figs 6a** and **6b,** the body weight of the pigs was monitored at all times from pre-immunization (3 weeks of age) to the end of this study (16 WPI, 24 weeks of age). The results revealed the influence of the vaccination over the animal's body weight. The pigs receiving saline as the negative controls and those receiving surgical castration as the positive controls both had significantly lower average body weight of 94.9 kg (negative controls, Groups 5 and 11) and 88.8 kg (surgical castration controls, Groups 6 and 12), respectively, at 24 weeks of age. In comparison to pigs receiving surgical castration (Groups 6 and 12), pigs receiving the vaccine formulations (Groups 4 and 10) of the current invention had a significantly greater economic benefit in body weight gain by about 20.3%.

### EXAMPLE 5

**Duration of Immunity for Pigs Receiving Varying Doses of LHRH1 (SEQ ID No: 10) Vaccine Formulations**

In this Example, the protocol was similar to the protocol of Example 3, except the peptide immunogen was changed to SEQ ID NO: 10, with dosage shown according to Table 7. After immunization, sera were collected at multiple time points for determination of titers of LHRH antibodies and the corresponding serum testosterone concentrations. Additionally, the sexual organs (testes and epididymides) of pigs were also collected upon sacrifice at 32 weeks of age for weight measurement.

The objective of this study was to extend the field trial to 24WPI, or 32 weeks of age, with serum samples collected for measurement of titers of LHRH antibodies and corresponding serum testosterone concentrations for assessment of the duration of the immunocastration efficiency of the vaccine formulations of the invention employing a standard immunization protocol with the prime at 8 weeks of age (0 WPI) and a boost at 16 weeks of age (8 WPI).

### a. Titration of Serum Anti-LHRH Antibodies by ELISA

As shown in **Fig. 7,** serum samples from pigs in Groups 1 to 4 were collected two weeks after the boost (i.e. 10 WPI) and assayed for titers of anti-LHRH antibodies. All samples were found to all have a titer more than 3.0 (logio) for anti-LHRH antibodies and such titers were sustained around 3.0 ($\log_{10}$) during a 10-weeks period until 20 WPI (i.e. 28 weeks of age). Moreover, Groups 2 and 3 maintained the anti-LHRH antibody titers at 3.0($\log_{10}$) until 24 WPI (i.e. 32 weeks of age). In contrast, the negative control group (Group 5) and castration control group (Group 6) always maintained a low profile background level antibody titer.

### b. Assessment of Serum Testosterone Concentration

As shown in **Fig. 8,** serum samples from pigs in Groups 1 to 4 were collected two weeks after the boost (i.e. 10 WPI) and assayed for serum testosterone concentration. All samples in pigs from Groups 1 to 4 were found to have serum testosterone concentrations at a castration level resulting from suppression by high titers of anti-LHRH antibodies.

The testosterone levels of Groups 1 to 4 maintained at as low as that of the surgical castration control group (Group 6) until 20 WPI (i.e. 28 weeks of age).

### c. Shrinkage of Sexual Organs (Testes and Epididymides) upon Immunocastration by LHRH1 Vaccine Formulations

At the end of the extended study at 24 WPI (i.e. pigs at 32 weeks of age), the pigs were sacrificed and their sexual organs including testes and epididymides were weighed for assessment of immunocastration efficiency. The testes and epididymides of the pigs from Groups 1 to 4 were found to shrink to malfunctioned sizes as shown in **Figs. 9-10.** The weights of testes and epididymides also correlated to the serum testosterone concentration.

### d. Body Weight

Pigs from Groups 1 to 4 had a better performance in body weight gain when compared to those pigs in the control groups (Groups 5 and 6) as shown in **Fig. 11.**

### e. Boar-Taint Removal as Measured by Concentrations of Androstenone and Skatole in Belly Fat

In this duration study, quantitation of boar-taint factors at the end of the study demonstrated that effective removal of boar taint in belly fat could prolong the pig sale window schedule to 24 WPI (i.e. 32 weeks of age). In this study, the androstenone and skatole were extracted from belly fat when the pigs were sacrificed and then detected by HPLC. According to **Fig. 12,** the concentrations of androstenone in fat were controlled to below 0.5 $\mu$g/g fat from pigs in Groups 1 to 4. Especially in Group 3, the mean value of androstenone was controlled down to 0.1384 $\mu$g/g fat. According to **Fig. 13,** the concentration of skatole was controlled to below 0.1 $\mu$g/g fat for pigs in Groups 1 to 3. For pigs in Group 3, the mean value of skatole was found to be the lowest at 0.0514 $\mu$g/g.

In addition, individual androstenone and skatole concentrations of a particular sample are plotted for boar-taint risk factor assessment and separated into four sectors as low, medium/low, medium/high, and high for all samples collected from this study as shown in **Fig. 14.** According to **Fig. 14,** the surgical castration group (Group 6) always maintained the lowest concentration of boar-taint factors. Samples from pigs in Group 3 had an excellent performance with all of the samples being distributed in the low risk sector until 24WPI. Samples from pigs in the Group 2 had good performance until 24 WPI when 7/8 of the samples were distributed in low risk with one sample in the high risk sector. Samples from pigs in Group 1 had a similar performance as those from Group 2 with 7/8 of the samples distributed in the low risk sector and one sample shown in the medium/high risk. In contrast, the saline control group had only 2/8 samples in the low risk sector, with 2/8 in the medium risk and 4/8 in the high risk sectors **(Fig. 14).** LHRH1 vaccine formulations of the invention demonstrated the boar-taint removal potency all the way until 24 WPI (i.e. 32 weeks of age). The results shown in this study indicated clearly that pigs receiving the LHRH1 vaccine formulations of the invention at varying doses can effectively suppress the production of androstenone and skatole, and thus remove boar-taint from the corresponding meat product(s).

### EXAMPLE 6

### Effect of Immunization Schedule on Immunocastration with Prime and Boost Conducted at Various Time Intervals with LHRH 1 Vaccine Formulations

In this Example, the protocol was similar to the protocol of Example 3, except that the peptide immunogen used in the vaccine formulations was changed to SEQ ID NO: 10 with adjuvant ISA50V2, and the time points of prime and boost immunization were modified according to Table 8. Sera were collected at various time points for measurement of titers of anti-LHRH antibodies and serum concentrations of testosterone.

As illustrated in **Fig. 15,** pigs in Group 1 demonstrated high immunogenicity with the mean antibody titer against LHRH measured at 3.556 (logio) for pigs at 22 weeks of age and such titers were maintained at 3.235 ($\log_{10}$) until 26 weeks of age. For pigs in Group 2, the highest antibody titer was found at 3.526 ($\log_{10}$) at 18 weeks of age and maintained at 2.869 ($\log_{10}$) at 26 weeks of age. For pigs in Group 3, due to the late boost immunization schedule at 18 weeks of age, the highest antibody titer was 3.219 ($\log_{10}$) at 20 weeks of age, two weeks after the boost, and the titer was maintained at 2.682 ($\log_{10}$) until 26 weeks of age.

Additionally, as illustrated in **Fig. 16,** pigs in Group 1 revealed an immunocastration effect (testosterone concentration $\leqq$ 1.0 nmol/L) at 20 weeks of age, i.e. two weeks after the boost with the immunocastration effect sustained for at least 6 weeks at 26 weeks of age. Pigs in Group 2 also revealed high immunocastration efficacy. The testosterone concentration of pigs from Group 2 was lower than 1.0 nmol/L during a 2 to 6 weeks period after the boost (i.e. at 22 weeks of age) and maintained at a relative low level (testosterone concentration $\leqq$ 1.644 nmol/L) until 26 weeks of age. Two weeks after the boost, the mean testosterone concentration in pigs from Group 3 was at 4.535 nmol/L. The testosterone concentration of pigs from Group 3 was suppressed to 1.968 nmol/L at 26 weeks old. It is beneficial for the farmers to initiate the vaccine priming in pigs at an earlier age, preferably at around 3 weeks of age, for ease of animal handling. The farmers could also boost the pigs at an older age thus providing a time buffer zone for the workers to immunize the pigs and ensure the testosterone concentration to be maintained steadily at a low castrating level for practicality in immunocastration operation at the farms. The results as shown in this study indicated that the vaccine formulations of the invention can be applied as early as 3 to 4 weeks old and as late for the boost as the age of 18 weeks. Both prime and boost schedules could induce high immunogenicity leading to effective suppression of serum testosterone concentration in pigs at the age of 22 to 26 weeks which is the usual window for pig sales on the market in the pork industry.

## EXAMPLE 7

### Boar-Taint Removal by LHRH3 Vaccine Formulations

A conservative and consumer-friendly approach was established in the pork industry to set the cut-off value for androstenone at 0.5 $\mu$g/g fat for boar taint risk factor assessment. The low risk for tainted meat could be obtained either with a medium androstenone level (0.5-1.0 $\mu$g/g fat) combined with a low skatole level (<0.1$\mu$g/g fat) or with a low level of androstenone (<0.5 $\mu$g/g fat) combined with a medium level of skatole (0.1-0.22 $\mu$g/g fat). A medium risk would occur when both parameters (biomarkers) for boar taint occur in medium levels (androstenone 0.5-1.0 $\mu$g/g fat; skatole 0.1-0.22 $\mu$g/g fat). When both biomarkers (androstenone and skatole) exceed these cut off values at the same time it would imply a high risk for the meat to be regarded as tainted (Table 9).

In this Example, the protocol was about the same as that of Example 3. Pigs were immunized with LHRH3 (SEQ ID NOs: 7, 8 and 9) formulated in adjuvant ISA50V2 to form the water-in-oil (W/O) emulsion as shown in Table 10. After immunization, sera were collected at multiple time points for measurement of serum testosterone concentrations and the titers of anti-LHRH antibodies. Additionally, the tissues and organs of pigs were collected for measurement of the weight of testes and epididymides, and for measurement of amount of skatole and androstenone in belly fat by HPLC.

### a. Shrinkage of Testes and Epididymides

The shrinkage of testes and epididymides was observed with the isolated organs weighted when the pigs were sacrificed at various time points , i.e. 10, 12, 14, and 16 WPI, respectively, of the study **(Figs. 17a-17d,** respectively). Upon immunization with the vaccine formulations (LHRH3) of the invention, the testes and epididymides were found to shrink over time under the suppressive influence of the increasing concentration of anti-LHRH antibodies during this study period with significant weight loss shown at 10 WPI for epididymides and at 12 WPI (p < 0.05) for testes as shown in **Fig. 17.**

### b. Measurement of Boar-Taint

5g of back fat collected from vaccinated pigs or intact boars was individually added to 1.5 mL of 100% methanol. The fat was then grounded with sand texture slides until the tissues were completely homogenized. The fat extract was transferred into 15 mL centrifuge tubes and sonicated for 5 minutes at room temperature. The fat extract was cooled on ice for 15 minutes, and then centrifuged at 4,000 rpm for 20 minutes at 5°C to separate from the tissue debris. The supernatant was transferred to another 1.5 ml centrifuge tube.

After immunization of pigs with the vaccine formulations, peak titer of anti-LHRH antibodies appeared at 10 WPI, i.e. 2 weeks after the boost, with the testosterone level being suppressed to near castration level around or lower than 1.0 nmol/L which yielded decreasing concentrations of the androstenone in fat as illustrated in **Fig. 18a.** The androstenone concentrations of pigs receiving LHRH3 vaccine formulations of the invention ranged from less than 0.02 to 0.53 $\mu$g/g fat in comparison to the androstenone concentrations of pigs from the negative control group receiving saline which was from approximately less than 0.02 to 2.12 $\mu$g/g fat. The androstenone concentration was significantly suppressed at 10 WPI in comparison to the control groups (p < 0.05) until the end of this study at 16 WPI (24 weeks of age).

Moreover, the skatole concentration of pigs receiving the LHRH3 vaccine formulations of the invention was from about 0.004 to 0.2 $\mu$g/g fat **(Fig. 19b)** when compared to the skatole concentration of pigs from the negative control group which was from approximately 0.04 to 0.36 $\mu$g/g fat **(Fig. 19a).** The skatole concentrations of pigs receiving the LHRH3 vaccine formulations of the invention were significantly decreased only at 14 WPI due to the fact that it is often controlled by the feed taken in by the pigs and metabolized into the derivatives such as skatole stored in the corresponding fat tissue. According to industry conclusion when both androstenone and skatole exceed the cut-off (androstenone: 0.5

μg/g fat; skatole: 0.22 μg/g fat) at the same time, it would imply a high risk for the meat to be regarded as tainted. The results as illustrated in **Fig. 19** indicated that all of the fat samples obtained from the vaccinated groups belong to the low risk sector in a sensitized assessment to the unpleasant odor with low androstenone (< 0.5 μg/g fat) and low skatole concentration (< 0.22 μg/g fat) either at 10 WPI or at 16 WPI; whereas the pigs in negative control group have about a 50% low quality of meat exposure to the risk of unpleasant aroma in cooking.

## EXAMPLE 8

**Flexibility in Immunization Scheme of the LHRH1 Vaccine Formulations of the Invention**

In this Example, the protocol was similar to the protocol of Example 3, with pigs immunized with the LHRH1 (SEQ ID NO: 10) peptide formulated in adjuvant ISA50V2 to form a water-in-oil (W/O) emulsion as shown in Table 11 except that the dosage was at 25ug/2mL/dose , and the time points of vaccination were modified according to Table 11.

### a. Titration of Serum Anti-LHRH Antibodies by ELISA

The titer of anti-LHRH antibodies at the initial stage of this study was $1.458 \pm 0.007$ ($Log_{10}$) (Mean $\pm$ SD), which was the background of the assay. After prime and boost immunizations (Groups 1 to 3), the antibody titers increased gradually and all reached the levels at 2 weeks post boost. The mean antibody titer of Group 1 declined from $3.636 \pm 0.577$ ($Log_{10}$) at the peak level at 10 WPI to $2.418 \pm 0.742$ ($Log_{10}$) at the end of the study. The mean antibody titer of Group 2 declined from $3.868 \pm 0.221$ ($Log_{10}$) at the highest level among these 4 groups to $2.806 \pm 0.213$ ($Log_{10}$) at the end of the study. The immune responses of Groups 3 and 4 were similar in antibody profiles. The results indicated that the 1st immunization could be administered from 8 weeks of age to 3 weeks of age, preferably **(Fig. 20)**.

### b. Assessment of Serum Testosterone Concentration

As illustrated in **Fig. 21,** the testosterone concentrations of all the groups at the initial stage of the experiment was $2.943 \pm 2.854$ (mean $\pm$ SD) nmol/L. The time point set for vaccine potency evaluation is at 2 weeks post boost, which usually indicated the lowest TT concentration in immunocastration. Based on this observation, the average testosterone concentration of Groups 1 to 4 were found to be $1.265 \pm 0.353$, $1.329 \pm 0.636$, $1.904 \pm 2.297$ and $1.222 \pm 0.445$ (mean $\pm$ SD) nmol/L, respectively. There was no statistically significant difference (p= 0.992) between groups, which was analyzed by Kruskal-Wallis One Way Analysis of Variance on Ranks. Additionally, the anti-LHRH antibody titers showed a reverse correlation with the testosterone concentrations with -0.72 correlation coefficient in which high titer of anti-LHRH antibodies caused low testosterone concentrations after boost.

For assessment of duration of immunity, pigs in Group 3 indicated the best performance (i.e. lowest testosterone concentrations) whose average serum testosterone concentration at 24 weeks of age was $0.896 \pm 0.386$ nmol/L. There was no significant difference (P = 0.835) between Groups 3 and 4. As the schedule advanced, the testosterone concentration increased to $1.913 \pm 0.930$ nmol/L (Group 2) and $4.149 \pm 3.603$ nmol/L (Group 1). There was a significant difference between Groups 1 and 3 (P = 0.010) as analyzed by Dunn's method.

### c. Measurement of Sexual Organs

The in-life testis sizes (length and width) were measured from 12 weeks of age on and were calculated into volume, using the formulae as follows:

$$\text{Testis volume} = 1/2 \text{ x a x b}^2,$$

where

"a" is the length of testis
"b" is the width of testis

The sexual organ measurements included (1) the testis in length and width in life, and (2) the testis and epididymis weights, length and width at necropsy. According to **Figs. 22** and 23, there was no difference in the weights of testis pair, epididymis pair and testis length between Groups 1 to 4. Both in-life testis length and volume **(Figs. 22a and 22b)** were highly suppressed by 22 weeks of age. However, in each parameter, pigs in the Group 2 had the best results.

The distribution of androstenone and skatole concentrations at 24 weeks of age was shown in **Fig. 24.** According to the favorable boar taint factor concentration category, 87.5% pigs of Groups 1, 3 and 4 were "low risk", which is not sensitive for human taste and smell. All pigs in Group 2 fell into the "low risk" zone thus 100% efficacious.

#### d. Body Weight

The body weight of each pig was also measured in this study. Each group of pigs grew in a similar pattern. The average weight for pig sales on the market in Taiwan is about 115 kg, which could be achieved by 26 weeks of age.

In conclusion, in comparison to the regular immunization program with prime and boost conducted at 8 and 16 weeks of age respectively, the prime schedule of this invention could be flexibly changed to the pig age of 3 weeks old. Furthermore, the boost schedule could be given as late as from age of 14 to 16 weeks as shown in this study.

#### EXAMPLE 9

#### Early Stage Flexibility Treatment Study with the LHRH1 Vaccine Formulation

In this Example, the protocol was similar to the protocol of Example 3, except that the peptide immunogen employed in the vaccine formulation was changed to SEQ ID NO: 10, and the time points of vaccination were modified according to Table 12. The purpose of this Example is to assess the duration of castration if the vaccine formulation is administrated to very young piglets and the immunological treatment results in sexual organ atrophy. This experiment is designed to evaluate very early "Flexible Treatment Regimens" and to determine the earliest prime time in the immunization regimen.

#### a. Titration of Serum Anti-LHRH Antibodies

As illustrated in **Fig. 25,** early immunization achieved a mean anti-LHRH antibody titer of $2.346 \pm 0.451$ $(Log_{10})$ for Group 1 at 12 weeks old, which was immunized at three days (D3) of age for the prime and at three weeks of age for the boost , and $2.173 \pm 0.527$ $(Log_{10})$ for Group 2 at 6 weeks of age, which was immunized at seven days of age for the prime and at three weeks of age for the boost **(Fig. 25).**

Of these groups, only pigs in Group 3 with the prime immunization at three weeks old and boost at 6 weeks of age mounted a high titer of anti-LHRH antibodies of $2.767 \pm 0.476$ $(Log_{10})$ at eight weeks of age. For pigs in Groups 1 to 3, after anti LHRH antibodies achieving the highest titers two weeks after the boost, the antibody titers were maintained at $1.718 \pm 0.297$, $1.765 \pm 0.340$ and $1.770 \pm 0.283$ $(Log_{10})$ for Group 1, 2, and 3, respectively. Pigs in Groups 3 and 4 also received prime immunization conducted at three weeks of age but boosted at 16 weeks of age. Pigs in Groups 4 and 5 could mount higher antibody titers, $3.249 \pm 0.346$ and $2.893 \pm 0.786$ at 18 weeks old respectively, two weeks after boost at the age of 16 weeks. As for pigs receiving the commercial vaccine Improvac® (Group 6), the titers of anti-LHRH antibodies were induced to $1.724 \pm 0.339$ $(Log_{10})$ at 20 weeks old, i.e. two weeks after the boost. Neither the positive control, i.e. pigs receiving surgical castration (Group 7), nor the negative control, i.e. pigs receiving no vaccination (Group 8), could induce the anti-LHRH antibodies titers beyond the background level, which was assayed with the value of $1.451 \pm 0.006$ and $1.445 \pm 0.010$ $(Log_{10})$ respectively.

#### b. Assessment of Serum Testosterone Concentration

As illustrated in **Fig. 26,** the serum testosterone level for pigs in Groups 1 and 2 declined to the lowest concentration of $2.154 \pm 0.921$ nmol/L at 10 weeks of age and $2.183 \pm 1.231$ nmol/L at 6 weeks of age, respectively. In Group 3 the first dose was administrated at three weeks of age, and the testosterone profile is similar to that of Groups 1 and 2. The lowest testosterone level of Group 3 was $3.065 \pm 0.205$ nmol/L at 12 weeks of age.

When the boost immunization was delayed to age of 16 weeks in Groups 4 and 5, regardless of whether the first dose was administrated at 3 or 8 weeks of age, the testosterone concentration was suppressed to low levels at around $0.586 \pm 0.184$ and $0.893 \pm 1.192$ nmol/L for Groups 4 and 5 at 20 weeks of age, respectively. In contrast, although pigs in Group 6 followed the immunization instruction of Improvac®, the testosterone level in pigs remained at high concentration (about $9.415 \pm 7.560$ nmol/L) in serum at 20 weeks of age. For pigs in Group 7 receiving surgical castration, the testosterone concentrations for each of the pigs always were maintained at background level (about $0.559 \pm 0.372$ nmol/L). As for pigs in Group 8 receiving no vaccination and served as negative controls, the mean testosterone concentration was maintained at normal concentration (between $3.546 \pm 2.409$ nmol/L and $7.380 \pm 3.269$ nmol/L) during the trial.

In conclusion, the anti-LHRH antibody titers could be induced efficiently with the prime immunization at as early as 3 weeks of age and a boost thereafter which maintained high titers of such antibodies at the end of the trial giving a similar antibody profile to those following a regular immunization scheme with prime immunization at 8 weeks of age and boost at 16 weeks of age. Therefore, the serum testosterone concentration could be suppressed to low levels efficiently when the prime immunization was administered at 3 weeks to 8 weeks of age while the boost was administered at 16 weeks of age. It is convenient for the farmers to conduct immunocastration with the flexible early stage immunization scheme for pigs at 3 weeks of age when many other vaccines in general are being administered.

## EXAMPLE 10

**Effective Immunocastration Demonstrated in a Field Trial Using LHRH3 Vaccine Formulation at 100 μg/mL/dose with Prime and Boost Immunizations Conducted at 8 and 16 Weeks of Age with Study Ending at 22 Weeks of Age.**

A large-scale field study was conducted in a pig farm located in southern Taiwan to assess the immunocastration efficacy in a field practice with two GMP batches of LHRH3 vaccine formulations. In this study, thirty-six crossbred male pigs and eight castrated pigs at 8 to 9 weeks of age were prepared in the farm.

Pigs were classified into 4 groups, and weighed at 0, 8, 10, 12, and 14 WPI. All pigs were immunized at 0 and 8 WPI and bled at 0, 8, 10, 12, and 14 WPI for serum titers of anti-LHRH antibodies and serum testosterone concentrations as shown in Table 13. After slaughter, weights of sexual organs (testes, epididymis, seminal vesicles, and prostate glands) with back fat thickness and loin-eye area (logissimus muscle) measured.

### a. Titration of Serum Anti-LHRH Antibodies by ELISA

As illustrated in **Fig. 27** and Table 14, the anti-LHRH antibody titers for pigs in Groups 1 and 2 were significantly higher than pigs in Groups 3 (intact control) and 4 (surgical castration control). For examples, the levels of anti-LHRH antibody titers were about $1.52 \pm 0.12$ ($Log_{10}$) to $2.90 \pm 0.42$ ($Log_{10}$) in Group 1, and $1.45 \pm 0.01$ ($Log_{10}$) to $3.09 \pm 0.37$ ($Log_{10}$) in Group 2. In comparison, all anti-LHRH antibody titer of Groups 3 and 4 was significantly lower than that of Groups 1 and 2.

### b. Assessment of Serum Testosterone Concentration for Immunocastration

As illustrated in **Fig. 28** and Table 15, all pigs in Groups 1 and 2 were castrated with a immunocastration rate at 100% after immunization of LHRH3 vaccine formulation at 100ug/mL/dose. The testosterone concentrations of all pigs in Groups 1 and 2 were suppressed to a very low level from 8 WPI on to achieve the immunocastration. The results demonstrated that the LHRH 3 vaccine formulation of the present invention could effectively inhibit characteristics of sexual maturation in pigs.

### c. Increase in Body Weights in Vaccinated Pigs

The body weight of each pig was measured as listed in Table 16. As observed, body weights of pigs in Groups 1 and 2 are heavier than that of Group 3.

As illustrated in **Fig. 29** and Table 17, the increased Average Daily Gain (ADG) of pigs was greater in vaccinated groups (Groups 1 and 2) when compared to the castrated control (Group 4). The increase in ADG was mainly due to increased Average Daily Feed Intake (ADFI), but not Food Efficiency (FE). The results in this field trial demonstrated that the LHRH3 vaccine formulation of the present invention when administered at 100 μg per dose in a prime and boost regimen at 8 and 16 weeks of age respectively was found highly effective to lead to immunocastration with significantly enhanced growth in male pigs. As a result of this enhanced growth, pigs could be sold on the market by more than two weeks ahead of the regular breeding schedule thus generating significant additional economic benefits to the farmers as well.

### d. Shrinkage of Genital Track Sexual Organs

At the end of this study at 14 WPI (i.e.22 weeks of age), the pigs were sacrificed and the testes, epididymides, seminal vesicles, and prostate gland were weighted for the evaluation of the immunocastration efficiency. For pigs in Groups 1 and 2, the weights of genital tract sexual organs including testes, epididymides, seminal vesicles, and prostate gland were found to be significantly decreased (Table 18), with these genital tract sexual organ shrinking to nonfunctional sizes.

In summary, the LHRH peptide immunogen based vaccine formulations of the present invention has a higher immunocastration effect than currently available commercial product (Improvac®). In addition to the humane factor for conversion of conventional surgical castration into the upcoming immunocastration in the animal husbandry field, the significant weight gains as a result of such immunocastration practice will generate significant economic benefits which would push the trend of animal castration to the front line after decades of exploration.

While the invention has been described by way of examples and in terms of the preferred embodiments, it is to be understood that the invention is not limited to the disclosed embodiments. To the contrary, it is intended to cover various modifications and similar arrangements (as would be apparent to those skilled in the art). Therefore, the scope of the appended claims should be accorded the broadest interpretation so as to encompass all such modifications and similar arrangements.

**Table 1**

| The Sequences of LHRH, Th Epitopes and a Co-stimulatory Invasin Peptide Domain | | |
|---|---|---|
| SEQ ID NO: | Peptide | Amino Acid Sequences |
| 1 | LHRH | EHWSYGLRPG |
| 2 | TT1 Th | KKQYIKANSKFIGLTEL |
| 3 | MVF Th | LSEIKGVIVHRLEGVGG |
| 4 | HBsAg Th | FFLLTRILTIPQSLE |
| 5 | SSAL1 Th | ISITEIRTVIVTRIETILF<br> S   KG    HK |
| 6 | Co-stimulatory peptide domain from Invasin | TAKSKKFPSYTATYQF |

**Table 2**

| The Sequences of the LHRH Peptide Immunogens of the Invention | |
|---|---|
| SEQ ID NO: | Amino Acid Sequences |
| 7 | KKQYIKANSKFIGITELEHWSYGLRPG |
| 8 | TAKSKKFPSYTATYQFGGLSEIKGVIVHRLEGVGGEHWSYGLRPG |
| 9 | TAKSKKFPSYTATYQFGGFFLLTRILTIPQSLEGGEHWSYGLRPG |
| 10 | TAKSKKFPSYTATYQF-εK-ISITEIRTVIVTRIETILF-εK-EHWSYGLRPG<br>                              S   KG    HK |

**Table 3**

| Study Design for the LHRH3 Formulation in ISA50V2 | | | | | | | |
|---|---|---|---|---|---|---|---|
| Group No. | Animal No. | Test Article | Dosage (conc. of peptide) | Admin. Route | 1st dose | 2nd dose | Sacrifice |
| 1 | 8 | LHRH3 §/ISA50V2 | 50 μg/1 mL/ dose | I.M. | 8* (0 WPI#) | 16* (8 WPI#) | 24* (16 WPI#) |
| 2 | 8 | LHRH3 §/ISA50V2 | 100 μg/ 1 mL/ dose | I.M. | 8* (0 WPI#) | 16* (8 WPI# ) | 24* (16 WPI#) |
| 3 | 8 | LHRH3 §/ISA50V2 | 150 μg/ 1 mL/ dose | I.M. | 8* (0 WPI#) | 16* (8 WPI#) | 24* (16 WPI# ) |
| 4 | 8 | LHRH3 §/ISA50V2 | 200 μg/ 1 mL/ dose | I.M. | 8* (0 WPI#) | 16* (8 WPI#) | 24* (16 WPI#) |
| 5 | 8 | Saline (Negative control) | 0 μg/ 2 mL/ dose | I.M. | 8* (0 WPI#) | 16* (8 WPI#) | 24* (16 WPI |
| 6 | 8 | None (Surgical Castration) | - | - | - | - | 24* |

(continued)

| Study Design for the LHRH3 Formulation in ISA50V2 | | | | | | | |
|---|---|---|---|---|---|---|---|
| Group No. | Animal No. | Test Article | Dosage (conc. of peptide) | Admin. Route | 1st dose | 2nd dose | Sacrifice |
| 7 | 8 | Improvac® | 300μg/ 2mL/ dose | S.C. | 12* (0 WPI#) | 20* (8 WPI#) | 24* (12 WPI) |

*Weeks of age
#WPI: **W**eeks **P**ost (first) Immunization
§LHRH3 contains the peptides of SEQ ID NOs:7, 8 and 9.

**Table 4**

| Study Design for the LHRH3 Formulation in Emulsigen D | | | | | | | |
|---|---|---|---|---|---|---|---|
| Group No. | Animal No. | Test Article | Dosage (conc. of peptide) | Admin Route | 1st dose | 2nd dose | Sacrifice |
| 8 | 8 | LHRH3 §/ Emulsigen D | 50 μg/ 2 mL/ dose | I.M. | 8* (0 WPI#) | 16* (8 WPI#) | 24* (16 WPI#) |
| 9 | 8 | LHRH3 §/ Emulsigen D | 100 μg/ 2 mL/ dose | I.M. | 8* (0 WPI#) | 16* (8 WPI#) | 24* (16 WPI#) |
| 10 | 8 | LHRH3 §/ Emulsigen D | 150 μg/ 2 mL/ dose | I.M. | 8* (0 WPI#) | 16* (8 WPI#) | 24* (16 WPI#) |
| 11 | 8 | LHRH3 §/ Emulsigen D | 200 μg/ 2 mL/ dose | I.M. | 8* (0 WPI#) | 16* (8 WPI#) | 24* (16 WPI#) |
| 12 | 8 | Saline (Negative control) | 0 μg/ 2 mL/ dose | I.M. | 8* (0 WPI#) | 16* (8 WPI#) | 24* (16 WPI) |
| 13 | 8 | None (Surgical castration) | - | - | - | - | 24* |
| 14 | 8 | Improvac® | 300μg/ 2mL/ dose | S.C. | 12* (0 WPI#) | 20* (8 WPI#) | 24* (12 WPI) |

*Weeks of age
#WPI: **W**eeks **P**ost (first) Immunization
§LHRH3 contains the peptides of SEQ ID NOs: 7, 8, and 9.

**Table 5**

| Study Design for LHRH1 Formulation in ISA50V2 | | | | | | | |
|---|---|---|---|---|---|---|---|
| Group No. | Animal No. | Test Article | Dosage (conc. of peptide) | Admin Route | 1st dose | 2nd dose | Sacrifice |
| 1 | 8 | LHRH1§/ISA50V2 | 12.5 μg/ 2 ml/ dose | I.M. | 8* (0 WPI#) | 16* (8 WPI#) | 24* (16 WPI#) |
| 2 | 8 | LHRH1§/ISA50V2 | 25 μg/ 2 ml/ dose | I.M. | 8* (0 WPI#) | 16* (8 WPI#) | 24* (16 WPI#) |
| 3 | 8 | LHRH1§/ISA50V2 | 50 μg/ 2 ml/ dose | I.M. | 8* (0 WPI#) | 16* (8 WPI#) | 24* (16 WPI#) |
| 4 | 8 | LHRH1§/ISA50V2 | 100 μg/ 2 ml/ dose | I.M. | 8* (0 WPI#) | 16* (8 WPI#) | 24* (16 WPI#) |

(continued)

| Study Design for LHRH1 Formulation in ISA50V2 | | | | | | | |
|---|---|---|---|---|---|---|---|
| Group No. | Animal No. | Test Article | Dosage (conc. of peptide) | Admin Route | 1st dose | 2nd dose | Sacrifice |
| 5 | 8 | Saline (Negative control) | 0 μg/ 2 mL/ dose | I.M. | 8* (0 WPI#) | 16* (8 WPI#) | 24* (16 WPI) |
| 6 | 8 | None (Surgical castration) | - | - | - | - | 24* |

*Weeks of age;
#WPI: **W**eeks **P**ost (first) **I**mmunization
§LHRH1 contains the peptide of SEQ ID NO: 10

**Table 6**

| Study Design for LHRH1 Formulation in Emulsigen D | | | | | | | |
|---|---|---|---|---|---|---|---|
| Group No. | Animal No. | Test Article | Dosage (conc. of peptide) | Admin Route | 1st dose | 2nd dose | Sacrifice |
| 7 | 8 | LHRH1§/ Emulsigen D | 12.5 μg/ 2 ml/ dose | I.M. | 8* (0 WPI#) | 16* (8 WPI#) | 24* (16 WPI#) |
| 8 | 8 | LHRH1§/ Emulsigen D | 25 μg/ 2 ml/ dose | I.M. | 8* (0 WPI#) | 16* (8 WPI#) | 24* (16 WPI#) |
| 9 | 8 | LHRH1§/ Emulsigen D | 50 μg/ 2 ml/ dose | I.M. | 8* (0 WPI#) | 16* (8 WPI#) | 24* (16 WPI#) |
| 10 | 8 | LHRH1§/Emulsigen D | 100 μg/ 2 ml/ dose | I.M. | 8* (0 WPI#) | 16* (8 WPI#) | 24* (16 WPI#) |
| 11 | 8 | Saline (Negative control) | 0 μg/ 2 mL/ dose | I.M. | 8* (0 WPI#) | 16* (8 WPI#) | 24* (16 WPI) |
| 12 | 8 | None (Surgical castration) | - | - | - | - | * 24* |

*Weeks of age;
#WPI: **W**eeks **P**ost (first) **I**mmunization
§LHRH1 contains the peptide of SEQ ID NO. 10

**Table 7**

| Study Design for the Duration of Immunity | | | | | | | |
|---|---|---|---|---|---|---|---|
| Group No. | Animal No. | Test Article | Dosage (conc. of peptide) | Admin Route | 1st dose | 2nd dose | Sacrifice |
| 1 | 8 | LHRH1§/ISA50V2 | 12.5 μg/ 2 ml/ dose | I.M. | 8* (0 WPI#) | 16* (8 WPI#) | 24* (16 WPI#) |
| 2 | 8 | LHRH1§/ISA50V2 | 25 μg/ 2 ml/ dose | I.M. | 8* (0 WPI#) | 16* (8 WPI#) | 24* (16 WPI#) |
| 3 | 8 | LHRH1§/ISA50V2 | 50 μg/ 2 ml/ dose | I.M. | 8* (0 WPI#) | 16* (8 WPI#) | 24* (16 WPI#) |

(continued)

| Study Design for the Duration of Immunity | | | | | | | |
|---|---|---|---|---|---|---|---|
| Group No. | Animal No. | Test Article | Dosage (conc. of peptide) | Admin Route | 1st dose | 2nd dose | Sacrifice |
| 4 | 8 | LHRH1§/Emulsigen D | 100 µg/ 2 ml/ dose | I.M. | 8* (0 WPI#) | 16* (8 WPI#) | 24* (16 WPI#) |
| 5 | 8 | Saline (Negative control) | 0 µg/ 2 mL/ dose | I.M. | 8* (0 WPI#) | 16* (8 WPI#) | 24* (16 WPI) |
| 6 | 8 | None (Surgical castration) | - | - | - | - | 24* |

*Weeks of age;
#WPI: **W**eeks **P**ost (first) **I**mmunization
§LHRH1 contains the peptide of SEQ ID NO: 10

**Table 8**

| Time Points of Vaccination | | | | | | | | | |
|---|---|---|---|---|---|---|---|---|---|
| Group | Boar Age (weeks) | 4 | 8 | 16 | 18 | 20 | 22 | 24 | 26 |
| G1 | WPI | 0 | 4 | 12 | 14 | 16 | 18 | 20 | 22 |
| | Vaccination | Prime; 1ml/IM | | | Boost; 2ml/IM | | | | |
| | Bleed | | B | B | B | B | B | B | B |
| G2 | WPI | | 0 | 8 | 10 | 12 | 14 | 16 | 18 |
| | Vaccination | | Prime; 1ml/IM | Boost; 2ml/IM | | | | | |
| | Bleed | | B | B | B | B | B | B | B |
| G3 | WPI | | | | 0 | 2 | 4 | 6 | 8 |
| | Vaccination | | | | Prime; 1ml/IM | | Boost; 2ml/IM | | |
| | Bleed | | B | B | B | B | B | B | B |

Vaccination: SEQ ID NO: 10 of 25 µg/ml
B: Bleeding

**Table 9**

| Assessment for Risk of Boar-Taint by a Combinatorial Cut-Off Values of Androstenone and Skatole | | | |
|---|---|---|---|
| Skatole ((µg/g) | Androstenone ((µg/g) | | |
| | Low < 0.5 | Medium 0.5-1.0 | High > 1.0 |
| Low < 0.1 | Low risk | Low risk | Medium risk |
| Medium 0.1-0.22 | Low risk | Medium risk | High risk |
| High >0.22 | Medium risk | High risk | High risk |

**Table 10**

| | | | | | | | | |
|---|---|---|---|---|---|---|---|---|
| **Study Design for Boar-Taint Removal** | | | | | | | | |
| Group No. | Animal No. | Test Article | Dosage | Admin Route | 1st dose | 2nd dose | Sacrifice* | |
| 1 | 8 | LHRH3 §/ISA50V2 | 100 μg/1 mL/ dose | I.M. | 0 WPI | 8 WPI | 10 WPI | |
| 2 | 8 | Saline (Negative control) | 0 μg/1 mL/dose | I.M. | 0 WPI | 8 WPI | 10 WPI | |
| 3 | 8 | LHRH3 §/ISA50V2 | 100 μg/1 mL/ dose | I.M. | 0 WPI | 8 WPI | 12 WPI | |
| 4 | 8 | Saline (Negative control) | 0 μg/1 mL/dose | I.M. | 0 WPI | 8 WPI | 12 WPI | |
| 5 | 8 | LHRH3 §/ISA50V2 | 100 μg/1 mL/ dose | I.M. | 0 WPI | 8 WPI | 14 WPI | |
| 6 | 8 | Saline (Negative control) | 0 μg/1 mL/dose | I.M. | 0 WPI | 8 WPI | 14 WPI | |
| 7 | 8 | LHRH3 §/ISA50V2 | 100 μg/1 mL/ dose | I.M. | 0 WPI | 8 WPI | 16 WPI | |
| 8 | 8 | Saline (Negative control) | 0 μg/1 mL/dose | I.M. | 0 WPI | 8 WPI | 16 WPI | |

*Weeks of age
§LHRH3 contains the peptides of SEQ ID NOs: 7, 8, and 9.

**Table 11**

| | | | | | | | |
|---|---|---|---|---|---|---|---|
| **The Schedule of Immunization Flexibility Study** | | | | | | | |
| Group No. | Animal No. | Test Article | Dosage | Admin Route | 1st dose | 2nd dose | Sacrifice* |
| 1 | 8 | LHRH1§/ISA50V2 | 25 μg/2 mL/dose | I.M. | 3* (0 WPI) | 12* 8 WPI | 24* 10 WPI |
| 2 | 8 | LHRH1§/ISA50V2 | 25 μg/2 mL/dose | I.M. | 3* 0 WPI | 14* 8 WPI | 24* 10 WPI |
| 3 | 8 | LHRH1§/ISA50V2 | 25 μg/2 mL/dose | I.M. | 3* 0 WPI | 16* 8 WPI | 24* 12 WPI |
| 4 | 8 | LHRH1§/ISA50V2 | 25 μg/2 mL/dose | I.M. | 8* 0 WPI | 16* 8 WPI | 24* 12 WPI |

**Table 12**

| | | | | | | | |
|---|---|---|---|---|---|---|---|
| **The Schedule of Early Age Flexibility Study** | | | | | | | |
| Group No. | Animal No. | Test Article | Dosage | Admin Route | 1st dose | 2nd dose | Sacrifice* |
| 1 | 8 | LHRH1§/ISA50V2 | 50 μg/ mL/dose | I.M. | D3† (0 WPI#) | W3* (2 WPI#) | W26* (25 WPI#) |
| 2 | 8 | LHRH1§/ISA50V2 | 50 μg/1 mL/ dose | I.M. | D7† (0 WPI#) | W3* (2 WPI#) | 26* (25 WPI#) |
| 3 | 8 | LHRH1§/ISA50V2 | 50 μg/1 mL/ dose | I.M. | W3* (0 WPI# ) | W6* (3 WPI#) | 26* (23 WP1#) |
| 4 | 8 | LHRH1§/ISA50V2 | 50 μg/1 mL/ dose | I.M. | W3* (0 WPI#) | W16* (13 WPI#) | 26* (23 WPI#) |

(continued)

| Group No. | Animal No. | Test Article | Dosage | Admin Route | 1st dose | 2nd dose | Sacrifice* |
|---|---|---|---|---|---|---|---|
| 5 | 8 | LHRH1§/ISA50V2 | 50 μg/1 mL/ dose | I.M. | *W8* (0 WPI# ) | W16* (8 WPI#) | 26* (18 WPI#) |
| 6 | 8 | Improvac® | 300 μg/2 mL/ dose | S.C. | W12* (0 WPI#) | W20* (8 WPI#) | 26* (14 WPI#) |
| 7 | 8 | None (Surgical castration) | - | - | - | - | W26* |
| 8 | 8 | None (Intact Boar) | - | - | - | - | W26* |

**The Schedule of Early Age Flexibility Study**

D: Days of age;
*W: Weeks of age;
#WPI: **W**eeks **P**ost (first) **I**mmunization.

**Table 13**

| Group | Animal number | Immunization |
|---|---|---|
| 1 | 12 | 1.0 mL of the LHRH3 (SEQ ID NOs: 1, 2, and 3; 100 μg/mL batch1) vaccine formulation with adjuvant ISA50V, with prime immunization at 8 weeks of age and boost immunization at 16 weeks of age. |
| 2 | 12 | 1.0 mL of the LHRH3 (SEQ ID NOs: 1, 2, and 3; 100 μg/mL, batch 2) vaccine formulation with adjuvant ISA50V, with prime immunization at 8 weeks of age and boost immunization at 16 weeks of age. |
| 3 | 12 | no castration and no vaccination (intact control) |
| 4 | 8 | no vaccination (surgical castration control) |

**Study Design for the Duration of Immunity**

**Table 14**

**Titration of Anti-LHRH Antibodies**

| Group | Anti-LHRH antibodies titer ($Log_{10}$) | | | | |
|---|---|---|---|---|---|
| | 0WPI | 8WPI | 10WPI | 12WPI | 14WPI |
| 1-1 | 1.45 | 2.48 | 2.82 | 2.68 | 2.64 |
| 1-2 | 1.46 | 1.76 | 2.62 | 2.30 | 2.35 |
| 1-3 | 1.46 | 1.79 | 2.56 | 2.33 | 2.50 |
| 1-4 | 1.46 | 1.63 | 2.42 | 2.37 | 1.94 |
| 1-5 | 1.45 | 1.65 | 2.78 | 2.40 | 2.03 |
| 1-6 | 1.44 | 2.15 | 3.00 | 2.80 | 2.71 |
| 1-7 | 1.41 | 1.52 | 2.38 | 2.04 | 1.95 |
| 1-8 | 1.46 | 1.91 | 3.15 | 2.95 | 2.68 |
| 1-9 | 1.72 | 2.61 | 3.31 | 3.19 | 3.04 |
| 1-10 | 1.74 | 1.88 | 2.67 | 2.40 | 1.96 |
| 1-11 | 1.62 | 1.83 | 3.25 | 3.36 | 3.18 |
| 1-12 | 1.62 | 3.37 | 3.78 | 3.68 | 3.51 |

(continued)

| Titration of Anti-LHRH Antibodies | | | | | |
|---|---|---|---|---|---|
| Group | Anti-LHRH antibodies titer (Log$_{10}$) | | | | |
| | 0WPI | 8WPI | 10WPI | 12WPI | 14WPI |
| M±SD | 1.52 ± 0.12 | 2.05 ± 0.53 | 2.90 ± 0.42 | 2.71 ± 0.50 | 2.54 ± 0.52 |
| 2-1 | 1.46 | 2.86 | 3.10 | 3.02 | 2.93 |
| 2-2 | 1.45 | 1.88 | 2.62 | 2.68 | 2.53 |
| 2-3 | 1.45 | 2.64 | 3.13 | 2.90 | 2.75 |
| 2-4 | 1.45 | 1.78 | 3.26 | 2.51 | 2.26 |
| 2-5 | 1.46 | 2.33 | 3.09 | 3.03 | 2.83 |
| 2-6 | 1.45 | 2.33 | 3.80 | 3.69 | 3.39 |
| 2-7 | 1.45 | 1.71 | 3.00 | 3.00 | 2.66 |
| 2-8 | 1.45 | 2.65 | 3.61 | 3.27 | 3.12 |
| 2-9 | 1.44 | 2.26 | 2.83 | 2.81 | 2.69 |
| 2-10 | 1.44 | 1.96 | 2.55 | 2.45 | 2.60 |
| 2-11 | 1.45 | 1.79 | 2.85 | 2.68 | 2.46 |
| 2-12 | 1.48 | 2.49 | 3.28 | 3.09 | 2.92 |
| M±SD | 1.45 ± 0.01 | 2.22 ± 0.39 | 3.09 ± 0.37 | 2.93 ± 0.34 | 2.76 ± 0.30 |
| 3-1 | 1.45 | 1.47 | 1.45 | 1.45 | 1.45 |
| 3-2 | 1.45 | 1.45 | 1.45 | 1.45 | 1.45 |
| 3-3 | 1.45 | 1.45 | 1.45 | 1.45 | 1.45 |
| 3-4 | 1.45 | 1.51 | 1.52 | 1.51 | 1.52 |
| 3-5 | 1.41 | 1.43 | 1.44 | 1.44 | 1.44 |
| 3-6 | 1.35 | 1.47 | 1.57 | 1.79 | 1.76 |
| 3-7 | 0.57 | 0.52 | 0.66 | 0.90 | 1.29 |
| 3-8 | 1.57 | 1.74 | 1.79 | 1.72 | 1.70 |
| 3-9 | 1.47 | 1.47 | 1.46 | 1.47 | 1.45 |
| 3-10 | 1.45 | 1.46 | 1.46 | 1.45 | 1.45 |
| 3-11 | 1.45 | 1.46 | 1.47 | 1.45 | 1.45 |
| 3-12 | 1.46 | 1.45 | 1.45 | 1.51 | 1.52 |
| M±SD | 1.38 ± 0.26 | 1.41 ± 0.29 | 1.43 ± 0.26 | 1.47 ± 0.21 | 1.49 ± 0.12 |
| 4-1 | 0.87 | 0.92 | 1.48 | 1.72 | 1.51 |
| 4-2 | 1.41 | 1.91 | 1.75 | 1.56 | 1.48 |
| 4-3 | 0.44 | 0.76 | 0.54 | 1.03 | 1.32 |
| 4-4 | 0.32 | 0.60 | 0.22 | 1.47 | 1.43 |
| 4-5 | 1.44 | 1.45 | 1.43 | 1.08 | 1.30 |
| 4-6 | 1.46 | 2.15 | 2.50 | 2.63 | 2.21 |
| 4-7 | 0.99 | 1.14 | 1.14 | 1.14 | 0.87 |
| 4-8 | 1.06 | 1.44 | 1.43 | 1.32 | 1.47 |
| M±SD | 1.00 ± 0.44 | 1.30 ± 0.55 | 1.31 ± 0.70 | 1.49 ± 0.52 | 1.45 ± 0.37 |

**Table 15**

| Testosterone Concentration and Immunocastration | | | | | | |
|---|---|---|---|---|---|---|
| Group | Testosterone (nmol/L) | | | | | Castration * |
| | 0WPI | 8WPI | 10WPI | 12WPI | 14WPI | |
| 1-1 | 0.4408 | 0.0006 | 0.0006 | 0.0006 | 0.0006 | Yes |
| 1-2 | 0.2264 | 1.7984 | 0.0006 | 0.0006 | 0.0006 | Yes |

(continued)

| Group | Testosterone (nmol/L) | | | | | Castration * |
|---|---|---|---|---|---|---|
| | 0WPI | 8WPI | 10WPI | 12WPI | 14WPI | |
| 1-3 | 0.0213 | 0.7012 | 0.0006 | 0.0006 | 0.0376 | Yes |
| 1-4 | 0.3680 | 5.9201 | 0.0006 | 0.0006 | 0.0010 | Yes |
| 1-5 | 1.6817 | 15.2310 | 0.0846 | 0.0484 | 0.0520 | Yes |
| 1-6 | 5.8101 | 1.0160 | 0.0007 | 0.1639 | 0.0004 | Yes |
| 1-7 | 12.4470 | 2.5403 | 0.0643 | 0.0765 | 0.0004 | Yes |
| 1-8 | 3.3602 | 0.0966 | 0.0007 | 0.0119 | 0.0004 | Yes |
| 1-9 | 5.0971 | 0.2303 | 0.1186 | 0.0687 | 0.0571 | Yes |
| 1-10 | 1.1304 | 5.6268 | 0.0127 | 0.0004 | 0.0614 | Yes |
| 1-11 | 7.9728 | 0.9356 | 0.0876 | 0.0010 | 0.0004 | Yes |
| 1-12 | 2.8615 | 0.0956 | 0.0004 | 0.0004 | 0.0004 | Yes |
| 2-1 | 1.3274 | 0.0006 | 0.0006 | 0.0006 | 0.0006 | Yes |
| 2-2 | 1.3219 | 0.0006 | 0.0006 | 0.0006 | 0.0006 | Yes |
| 2-3 | 0.0495 | 0.0006 | 0.0006 | 0.0006 | 0.0006 | Yes |
| 2-4 | 0.1574 | 0.4006 | 0.0006 | 0.0006 | 0.0006 | Yes |
| 2-5 | 10.0500 | 17.3420 | 0.0007 | 0.2185 | 0.0081 | Yes |
| 2-6 | 6.1455 | 0.0007 | 0.0007 | 0.0007 | 0.0004 | Yes |
| 2-7 | 7.2511 | 5.8951 | 0.0065 | 0.2479 | 0.0013 | Yes |
| 2-8 | 2.2776 | 1.1869 | 0.0007 | 0.1506 | 0.0176 | Yes |
| 2-9 | 10.2550 | 3.8971 | 0.0689 | 0.0106 | 0.0004 | Yes |
| 2-10 | 13.3430 | 3.6233 | 0.0004 | 0.0004 | 0.0665 | Yes |
| 2-11 | 7.3201 | 2.9919 | 0.0454 | 0.0004 | 0.0004 | Yes |
| 2-12 | 17.7590 | 0.3083 | 0.0953 | 0.0004 | 0.0004 | Yes |
| 3-1 | 0.3934 | 19.3100 | 25.1850 | 15.6320 | 33.8750 | Intact |
| 3-2 | 0.0007 | 0.1349 | 4.0030 | 0.3542 | 10.0370 | Intact |
| 3-3 | 0.0007 | 6.5169 | 14.0690 | 5.0808 | 5.9247 | Intact |
| 3-4 | 0.1271 | 2.6575 | 4.8369 | 13.9270 | 10.4060 | Intact |
| 3-5 | 5.8650 | 9.5795 | 8.2874 | 15.9380 | 13.9300 | Intact |
| 3-6 | 5.0575 | 4.9353 | 0.5229 | 5.6140 | 5.3891 | Intact |
| 3-7 | 7.9479 | 11.9320 | 10.4350 | 17.1210 | 15.9700 | Intact |
| 3-8 | 3.7450 | 6.4745 | 16.0420 | 34.9350 | 32.4980 | Intact |
| 3-9 | 0.1573 | 6.0010 | 11.9220 | 2.9319 | 9.7829 | Intact |
| 3-10 | 4.4569 | 25.6050 | 34.4680 | 13.1240 | 19.9180 | Intact |
| 3-11 | 1.4491 | 8.0302 | 5.4376 | 5.9930 | 14.6140 | Intact |
| 3-12 | 0.9190 | 8.5474 | 15.7720 | 15.7810 | 23.7200 | Intact |
| 4-1 | 0.0004 | 0.1986 | 0.0004 | 0.0004 | 0.0004 | Surgical* |
| 4-2 | 0.0004 | 0.0004 | 0.0004 | 0.0004 | 0.0004 | Surgical* |
| 4-3 | 0.0049 | 0.0004 | 0.0004 | 0.0004 | 0.0004 | Surgical* |
| 4-4 | 0.0004 | 0.0004 | 0.0002 | 0.0069 | 0.0004 | Surgical* |
| 4-5 | 0.0198 | 0.0004 | 0.0004 | 0.0004 | 0.0004 | Surgical* |
| 4-6 | 0.0309 | 0.0004 | 0.0004 | 0.0004 | 0.0004 | Surgical* |
| 4-7 | 0.0004 | 0.0004 | 0.0004 | 0.0004 | 0.0004 | Surgical* |
| 4-8 | 0.0215 | 0.0004 | 0.0004 | 0.0004 | 0.0004 | Surgical* |

*The castration level for testosterone is set at $\leqq 1.87$ nmol/L (99% confidence interval) two weeks after second injection (10 wpi).

**Table 16**

| Body Weights of Pigs Receiving Vaccine Formulations (Groups 1 and 2) Compared to Those in the Control (Groups 3 and 4) | | | |
|---|---|---|---|
| Group | Pig No. | Initial BW (kg) | Final BW(kg) |
| 1-1 | 285-02 | 27.7 | 113.1 |
| 1-2 | 286-05 | 27.6 | 120.0 |
| 1-3 | 287-02 | 24.8 | 117.3 |
| 1-4 | 287-06 | 22.6 | 110.5 |
| 1-5 | 304-02 | 32.2 | 134.0 |
| 1-6 | 314-06 | 32.3 | 132.5 |
| 1-7 | 308-03 | 29.8 | 125.1 |
| 1-8 | 314-02 | 26.4 | 107.0 |
| 1-9 | 326-04 | 34.2 | 127.0 |
| 1-10 | 329-01 | 30.7 | 124.0 |
| 1-11 | 326-02 | 27.7 | 103.8 |
| 1-12 | 331-03 | 26.7 | 113.7 |
|  | M±SD | 28.6 ± 3.4 | 119.0 ± 9.8 |
| 2-1 | 287-03 | 28.3 | 127.5 |
| 2-2 | 288-01 | 26.9 | 147.8 |
| 2-3 | 286-02 | 23.7 | 110 |
| 2-4 | 288-02 | 26.6 | 112 |
| 2-5 | 304-04 | 33.2 | 149 |
| 2-6 | 304-05 | 31.7 | 114.1 |
| 2-7 | 308-02 | 28.7 | 131.1 |
| 2-8 | 314-04 | 26.6 | 124.3 |
| 2-9 | 331-02 | 32.2 | 136 |
| 2-10 | 323-05 | Cull |  |
| 2-11 | 326-03 | 28.6 | 112.2 |
| 2-12 | 331-01 | 27.3 | 129 |
|  | M±SD | 28.5 ± 2.8 | 126.6 ± 13.9 |
| 3-1 | 288-05 | 28.2 | 114.2 |
| 3-2 | 287-01 | 26.0 | 130.0 |
| 3-3 | 287-05 | 26.2 | 120.2 |
| 3-4 | 285-03 | 25.0 | 111.6 |
| 3-5 | 290-03 | 27.5 | 114.5 |
| 3-6 | 285-04 | 26.6 | 115.5 |
| 3-7 | 286-03 | 26.6 | 120.5 |
| 3-8 | 286-01 | 30.4 | 120.3 |
| 3-9 | 294-03 | 26.7 | 120.0 |
| 3-10 | 299-04 | 26.7 | 116.3 |
| 3-11 | 300-03 | 24.6 | 86.3 |
| 3-12 | 304-03 | 26.5 | 116.2 |
|  | M±SD | 26.8 ± 1.5 | 115.5 ± 10.3 |
| 4-1 | 352-02 | 29.4 | 100.0 |
| 4-2 | 346-03 | 31.5 | 97.0 |
| 4-3 | 351-04 | 32.4 | 123.5 |
| 4-4 | 352-07 | 30.4 | 121.6 |
| 4-5 | 352-03 | 31.2 | 115.7 |
| 4-6 | 346-01 | 30.4 | 111.5 |

(continued)

| Body Weights of Pigs Receiving Vaccine Formulations (Groups 1 and 2) Compared to Those in the Control (Groups 3 and 4) | | | |
|---|---|---|---|
| Group | Pig No. | Initial BW (kg) | Final BW(kg) |
| 4-7 | 351-01 | 32.2 | 111.7 |
| 4-8 | 352-05 | 28.2 | 99.0 |
| | M±SD | 30.7 ± 1.4 | 110.0 ± 10.3 |

**Table 17**

| Average Daily Gain, Average Daily Feed Intake, and Feed Efficiency | | | |
|---|---|---|---|
| Group | Average Daily Gain (kg) | Average Daily Feed Intake (kg) | Feed Efficiency |
| 1 | 0.923 | 2.489 | 2.696 |
| 2 | 1.000 | 2.632 | 2.632 |
| 3 | 0.906 | 2.220 | 2.453 |
| 4 | 0.809 | 2.288 | 2.828 |

**Table 18**

| Weight of Genital Tract Sexual Organs | | | | | |
|---|---|---|---|---|---|
| Group | Pig No. | Testes (g) | Epididymas (g) | Seminal Vesicles (g) | Prostate Gland (g) |
| 1-1 | 285-02 | 54.5 | 49.9 | 6.0 | 1.3 |
| 1-2 | 286-05 | 46.8 | 38.9 | 6.0 | 1.1 |
| 1-3 | 287-02 | 36.4 | 39.3 | 4.0 | 0.9 |
| 1-4 | 287-06 | 53.3 | 40.6 | 2.9 | 0.7 |
| 1-5 | 304-02 | 179.6 | 47.5 | 12.7 | 2.1 |
| 1-6 | 314-06 | 38.9 | 27.5 | 7.5 | 0.9 |
| 1-7 | 308-03 | 144.1 | 79.4 | 8.0 | 1.2 |
| 1-8 | 314-02 | 68.4 | 43.3 | 8.9 | 1.6 |
| 1-9 | 326-04 | 131.8 | 66.5 | 17.9 | 2.0 |
| 1-10 | 329-01 | 107.8 | 64.1 | 18.7 | 1.1 |
| 1-11 | 326-02 | 110.8 | 44.2 | 10.4 | 2.3 |
| 1-12 | 331-03 | 73.5 | 52.5 | 6.2 | 2.2 |
| | M±SD | 87.2 ± 46.8 | 49.5 ± 14.3 | 9.1 ± 5.0 | 1.5 ± 0.6 |
| 2-1 | 287-03 | 55.8 | 38.4 | 4.8 | 0.8 |
| 2-2 | 288-01 | 80.2 | 45.1 | 8.9 | 0.7 |
| 2-3 | 286-02 | 90.0 | 34.5 | 3.4 | 0.8 |
| 2-4 | 288-02 | 110.8 | 53.7 | 11.8 | 1.2 |
| 2-5 | 304-04 | 196.0 | 76.1 | 15.4 | 2.6 |
| 2-6 | 304-05 | 95.6 | 36.8 | 5.8 | 1.2 |
| 2-7 | 308-02 | 202.0 | 77.2 | 9.9 | 1.0 |
| 2-8 | 314-04 | 40.2 | 28.8 | 5.1 | 1.0 |
| 2-9 | 331-02 | 123.0 | 73.7 | 10.2 | 2.3 |
| 2-10 | 323-05 | | | | |
| 2-11 | 326-03 | 56.0 | 51.3 | 8.2 | 2.4 |
| 2-12 | 331-01 | 41.1 | 41.0 | 3.8 | 1.2 |

(continued)

|  | M±SD | 99.2 ± 50.3 | 50.6 ± 17.6 | 7.9 ± 3.8 | 1.4 ± 0.7 |
|---|---|---|---|---|---|
| 3-1 | 288-05 | 511.0 | 122.6 | 262.0 | 6.6 |
| 3-2 | 287-01 | 338.0 | 119.8 | 62.8 | 10.6 |
| 3-3 | 287-05 | 615.0 | 105.5 | 134.3 | 6.3 |
| 3-4 | 285-03 | 459.0 | 121.5 | 135.2 | 7.2 |
| 3-5 | 290-03 | 348.0 | 96.4 | 203.0 | 8.3 |
| 3-6 | 285-04 | 284.0 | 91.4 | 165.9 | 9.6 |
| 3-7 | 286-03 | 511.0 | 122.6 | 262.0 | 6.6 |
| 3-8 | 286-01 | 477.0 | 159.0 | 196.0 | 9.8 |
| 3-9 | 294-03 | 568.0 | 104.0 | 209.0 | 7.4 |
| 3-10 | 299-04 | 474.0 | 128.4 | 269.0 | 6.2 |
| 3-11 | 300-03 | 240.0 | 67.0 | 113.7 | 3.6 |
| 3-12 | 304-03 | 463.0 | 118.2 | 127.0 | 10.6 |
|  | M±SD | 463.9 ± 126.7 | 118.2 ± 24.1 | 170.5 ± 70.3 | 7.4 ± 1.9 |

**Claims**

1. A veterinary composition for immunocastration of pigs consisting of:

   (a) an effective amount of an equal molar mixture of peptide immunogens having an amino acid sequence represented by SEQ ID NOs: 7, 8, and 9 in a saline solution; and
   (b) a veterinarily acceptable delivery vehicle or adjuvant selected from the group consisting of: water, saline, alum, incomplete Freund's adjuvant (IFA), liposyn, saponin, squalene, L121, emulsigen, monophosphoryl lipid A (MPL), QS21, ISA 720, ISA50, ISA50V2, Emulsigen D, ISA 35, ISA 206, oils, lipids, polysorbate 80, emulsifiers, ethanol, polyol, and any combination thereof.

2. The veterinary composition according to claim 1, wherein the veterinarily acceptable delivery vehicle or adjuvant is ISA50V2.

3. The composition according to claim 1, wherein the veterinarily acceptable adjuvant is ISA50, ISA50V2 or Emulsigen D.

4. The composition according to claim 1, wherein the saline solution is 20% w/v NaCl.

5. The composition according to claim 1, wherein the total amount of the peptide immunogen is between 12.5 $\mu$g to 200 $\mu$g per dose.

6. The composition according to claim 1, wherein the veterinary composition is formulated into a 1 mL dosage unit containing:

   (a) a total of 100 $\mu$g of the equal molar mixture of peptide immunogens in 20% w/v NaCl saline solution; and
   (b) ISA50V2 as the veterinarily acceptable adjuvant.

7. A method for immunocastrating a pig comprising administering an effective amount of the composition according to any one of claims 1 to 6 to the pig.

8. The method according to claim 7, wherein the pig has reduced boar taint, sexual activity, sexuality, fertility, and estrous behavior after administering the composition.

9. The method according to claim 7, wherein the composition is administered by intramuscular or subcutaneous injection.

10. The method according to claim 7, wherein a first dose of the composition is applied to the pig at the age of 3 to 8 weeks old.

11. The method according to claim 10, wherein a second dose of the composition is applied to the pig at the age of 6 to 16 weeks old.

12. A method for reducing the production of testosterone in an animal comprising administering an effective amount of the composition according to any one of claims 1 to 6 to the animal.

13. A method for inducing antibodies against Luteinizing-hormone releasing hormone (LHRH) in a pig, comprising administering an effective amount of the composition according to any one of claims 1 to 6 to the pig.

**Fig. 1a**

**Fig. 1b**

**Fig. 2a**

**Fig. 2b**

**Fig. 3a**

Fig. 3b

Fig. 4a

**Fig. 4b**

**Fig. 5a**

**Fig. 5b**

**Fig. 6a**

**Fig. 6b**

**Fig. 7**

**Fig. 8**

*: Significantly different from G5 (Saline).

Fig. 9

Fig. 10

Fig. 11

**Fig. 12**

**Fig. 13**

**Fig. 14**

**Fig. 15**

**Fig. 16**

**Fig. 17a**

Fig. 17b

Fig. 17c

**Fig. 17d**

Vaccination, n=8
Entire Boars, n=8

Fig. 18a

**Fig. 18b**

Fig. 18c

Fig. 18d

**Fig. 19a**

**Fig. 19b**

**Fig. 20**

**Fig. 21**

# Mean Testis Length (In-life)

**Fig. 22a**

Mean Testis Volume* (In-life)

*: The mean testis volume is calculated as follows:
Testis volume = 1/2 x Length x (Width)$^2$

**Fig. 22b**

**Weight of Testes Pair (g)**

**Fig. 23a**

Weight of epididymis pair (g)

Fig. 23b

## Mean testis length (mm)

Fig. 23c

**Fig. 24**

**Fig. 25**

**Fig. 26**

**Fig. 27**

**Fig. 28**

Fig. 29

**EUROPEAN SEARCH REPORT**

Application Number

EP 20 21 4150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/009897 A1 (SOKOLL KENNETH K [US]) 15 January 2004 (2004-01-15) * paragraphs [0229], [0233], [0394], [0121], [0353], [0415], [0421], [0210], [0405], [0416] * ----- | 1-13 | INV. A61K39/00 A61K39/39 A61P15/00 |
| X | WO 94/25060 A1 (LADD ANNA E [US]; WANG CHANG YI [US]; ZAMB TIMOTHY [US]) 10 November 1994 (1994-11-10) * figures 21-35 * * Examples * ----- | 1-13 | |
| X | WO 03/068169 A2 (UNITED BIOMEDICAL INC [US]) 21 August 2003 (2003-08-21) * Examples * ----- | 1-13 | |
| A | WO 02/051860 A2 (UNITED BIOMEDICAL INC [US]) 4 July 2002 (2002-07-04) * Examples * ----- | 1-13 | |
| | | | TECHNICAL FIELDS SEARCHED (IPC) A61K |

The present search report has been drawn up for all claims

| Place of search | Date of completion of the search | Examiner |
|---|---|---|
| Munich | 18 May 2021 | Irion, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 20 21 4150

This annex lists the patent family members relating to the patent documents cited in  the above-mentioned European search report.
The members are as contained in the European Patent Office EDP file on
The European Patent Office is in no way liable for these particulars which are merely given for the purpose of information.

18-05-2021

| Patent document cited in search report | | Publication date | Patent family member(s) | | Publication date |
|---|---|---|---|---|---|
| US 2004009897 | A1 | 15-01-2004 | AT | 540697 T | 15-01-2012 |
| | | | AU | 2003213091 A1 | 04-09-2003 |
| | | | BR | 0307724 A | 27-04-2007 |
| | | | BR | PI0307724 B1 | 18-06-2019 |
| | | | CA | 2475102 A1 | 21-08-2003 |
| | | | CN | 101001646 A | 18-07-2007 |
| | | | DK | 1572074 T3 | 07-05-2012 |
| | | | EP | 1572074 A2 | 14-09-2005 |
| | | | ES | 2383172 T3 | 18-06-2012 |
| | | | HK | 1085652 A1 | 01-09-2006 |
| | | | JP | 5156170 B2 | 06-03-2013 |
| | | | JP | 5237997 B2 | 17-07-2013 |
| | | | JP | 2005530690 A | 13-10-2005 |
| | | | JP | 2010265291 A | 25-11-2010 |
| | | | MX | PA04007935 A | 08-06-2005 |
| | | | TW | I359025 B | 01-03-2012 |
| | | | US | 2003165478 A1 | 04-09-2003 |
| | | | US | 2004009897 A1 | 15-01-2004 |
| | | | WO | 03068169 A2 | 21-08-2003 |
| WO 9425060 | A1 | 10-11-1994 | AT | 221387 T | 15-08-2002 |
| | | | AU | 687805 B2 | 05-03-1998 |
| | | | CA | 2161445 A1 | 10-11-1994 |
| | | | DE | 69431114 T2 | 13-03-2003 |
| | | | DK | 0708656 T3 | 02-12-2002 |
| | | | EP | 0708656 A1 | 01-05-1996 |
| | | | ES | 2180576 T3 | 16-02-2003 |
| | | | FI | 955101 A | 21-12-1995 |
| | | | JP | 3795914 B2 | 12-07-2006 |
| | | | JP | H09503742 A | 15-04-1997 |
| | | | JP | 2005060406 A | 10-03-2005 |
| | | | NO | 316121 B1 | 15-12-2003 |
| | | | WO | 9425060 A1 | 10-11-1994 |
| WO 03068169 | A2 | 21-08-2003 | AT | 540697 T | 15-01-2012 |
| | | | AU | 2003213091 A1 | 04-09-2003 |
| | | | BR | 0307724 A | 27-04-2007 |
| | | | BR | PI0307724 B1 | 18-06-2019 |
| | | | CA | 2475102 A1 | 21-08-2003 |
| | | | CN | 101001646 A | 18-07-2007 |
| | | | DK | 1572074 T3 | 07-05-2012 |
| | | | EP | 1572074 A2 | 14-09-2005 |
| | | | ES | 2383172 T3 | 18-06-2012 |
| | | | HK | 1085652 A1 | 01-09-2006 |
| | | | JP | 5156170 B2 | 06-03-2013 |
| | | | JP | 5237997 B2 | 17-07-2013 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 20 21 4150

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2021

| Patent document cited in search report | Publication date | Patent family member(s) | | Publication date |
|---|---|---|---|---|
| | | JP | 2005530690 A | 13-10-2005 |
| | | JP | 2010265291 A | 25-11-2010 |
| | | MX | PA04007935 A | 08-06-2005 |
| | | TW | I359025 B | 01-03-2012 |
| | | US | 2003165478 A1 | 04-09-2003 |
| | | US | 2004009897 A1 | 15-01-2004 |
| | | WO | 03068169 A2 | 21-08-2003 |
| WO 02051860 A2 | 04-07-2002 | AR | 036006 A1 | 04-08-2004 |
| | | AU | 2002232917 A1 | 08-07-2002 |
| | | TW | I254050 B | 01-05-2006 |
| | | US | 2003027979 A1 | 06-02-2003 |
| | | US | 2004141993 A1 | 22-07-2004 |
| | | WO | 02051860 A2 | 04-07-2002 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9511998 A
- US 5759551 A
- US 6025468 A
- US 6228987 B
- US 6559282 B
- US 6713301 B

### Non-patent literature cited in the description

- **MEISTER et al.** Two novel T cell epitope prediction algorithms based on MHC-binding motifs; comparison of predicted and published epitopes from Mycobacterium tuberculosis and HIV protein sequences. *Vaccine,* vol. 13 (6), 581-591
- Synthetic Peptides: A Users Guide. WH Freeman and Company, 1992
- **MEISTER et al.** *Vaccine,* 1995, vol. 13, 581-591
- Synthetic Peptides: A User's Guide. W.H. Freeman & Co, 1992, 382

SEQUENCE LISTING

<110>  United Biomedical, Inc.

<120>  Immunogenic LHRH composition and use thereof in pigs

<130>  63260P EP-WO-1

<140>  TBD
<141>  2020-12-15

<160>  12

<170>  PatentIn version 3.5

<210>  1
<211>  10
<212>  PRT
<213>  Sus scrofa


<220>
<221>  PEPTIDE
<222>  (1)..(10)
<223>  LHRH peptide

<400>  1

Glu His Trp Ser Tyr Gly Leu Arg Pro Gly
1               5                   10


<210>  2
<211>  17
<212>  PRT
<213>  Sus scrofa


<220>
<221>  PEPTIDE
<222>  (1)..(17)
<223>  Tetanus toxin (aa. 830-844)

<400>  2

Lys Lys Gln Tyr Ile Lys Ala Asn Ser Lys Phe Ile Gly Leu Thr Glu
1               5                   10                  15

Leu


<210>  3
<211>  17
<212>  PRT
<213>  Sus scrofa


<220>
<221>  PEPTIDE
<222>  (1)..(17)
<223>  MVF Th

<400> 3

```
Leu Ser Glu Ile Lys Gly Val Ile Val His Arg Leu Glu Gly Val Gly
1               5                   10                  15

Gly
```

<210> 4
<211> 15
<212> PRT
<213> Sus scrofa

<220>
<221> PEPTIDE
<222> (1)..(15)
<223> HBsAg Th

<400> 4

```
Phe Phe Leu Leu Thr Arg Ile Leu Thr Ile Pro Gln Ser Leu Glu
1               5                   10                  15
```

<210> 5
<211> 19
<212> PRT
<213> Sus scrofa

<220>
<221> PEPTIDE
<222> (1)..(19)
<223> SSAL1 Th

<220>
<221> MISC_FEATURE
<222> (4)..(4)
<223> S or T

<220>
<221> MISC_FEATURE
<222> (7)..(7)
<223> K or R

<220>
<221> MISC_FEATURE
<222> (8)..(8)
<223> G or T

<220>
<221> MISC_FEATURE
<222> (12)..(12)
<223> H or T

<220>
<221> MISC_FEATURE
<222> (13)..(13)
<223> K or R

<400> 5

Ile Ser Ile Xaa Glu Ile Xaa Xaa Val Ile Val Xaa Xaa Ile Glu Thr
1               5                   10                  15

Ile Leu Phe


<210>   6
<211>   16
<212>   PRT
<213>   Sus scrofa


<220>
<221>   PEPTIDE
<222>   (1)..(16)
<223>   co-stimulatory peptide domain from Invasin

<400>   6

Thr Ala Lys Ser Lys Lys Phe Pro Ser Tyr Thr Ala Thr Tyr Gln Phe
1               5                   10                  15


<210>   7
<211>   27
<212>   PRT
<213>   Sus scrofa


<220>
<221>   PEPTIDE
<222>   (1)..(17)
<223>   Tetanus toxin Th

<220>
<221>   PEPTIDE
<222>   (18)..(27)
<223>   LHRH

<400>   7

Lys Lys Gln Tyr Ile Lys Ala Asn Ser Lys Phe Ile Gly Ile Thr Glu
1               5                   10                  15

Leu Glu His Trp Ser Tyr Gly Leu Arg Pro Gly
            20                  25


<210>   8
<211>   45
<212>   PRT
<213>   Sus scrofa


<220>
<221>   PEPTIDE
<222>   (1)..(16)

<223>    Co-stimulatory peptide domainfrom invasin

<220>
<221>    PEPTIDE
<222>    (17)..(18)
<223>    linker

<220>
<221>    PEPTIDE
<222>    (19)..(35)
<223>    MVF Th

<220>
<221>    PEPTIDE
<222>    (36)..(45)
<223>    LHRH

<400>    8

Thr Ala Lys Ser Lys Lys Phe Pro Ser Tyr Thr Ala Thr Tyr Gln Phe
1               5                   10                  15


Gly Gly Leu Ser Glu Ile Lys Gly Val Ile Val His Arg Leu Glu Gly
            20                  25                  30


Val Gly Gly Glu His Trp Ser Tyr Gly Leu Arg Pro Gly
            35                  40                  45


<210>    9
<211>    45
<212>    PRT
<213>    Sus scrofa


<220>
<221>    PEPTIDE
<222>    (1)..(16)
<223>    Co-stimulatory peptide domain from invasin

<220>
<221>    PEPTIDE
<222>    (17)..(18)
<223>    linker

<220>
<221>    PEPTIDE
<222>    (19)..(33)
<223>    HBsAg Th

<220>
<221>    PEPTIDE
<222>    (34)..(35)
<223>    linker

<220>
<221>    PEPTIDE
<222>    (36)..(45)
<223>    LHRH

<400>    9

Thr Ala Lys Ser Lys Lys Phe Pro Ser Tyr Thr Ala Thr Tyr Gln Phe
1               5               10                  15

Gly Gly Phe Phe Leu Leu Thr Arg Ile Leu Thr Ile Pro Gln Ser Leu
            20                  25                  30

Glu Gly Gly Glu His Trp Ser Tyr Gly Leu Arg Pro Gly
        35                  40                  45


<210>    10
<211>    47
<212>    PRT
<213>    Sus scrofa


<220>
<221>    PEPTIDE
<222>    (1)..(16)
<223>    Co-stimulatory peptide domain from invasin

<220>
<221>    PEPTIDE
<222>    (17)..(17)
<223>    epsilon K as a linker

<220>
<221>    PEPTIDE
<222>    (18)..(36)
<223>    SSAL1 Th

<220>
<221>    MISC_FEATURE
<222>    (21)..(21)
<223>    S or T

<220>
<221>    MISC_FEATURE
<222>    (24)..(24)
<223>    R or K

<220>
<221>    MISC_FEATURE
<222>    (25)..(25)
<223>    T or G

<220>
<221>    MISC_FEATURE
<222>    (29)..(29)
<223>    T or H

<220>
<221>    MISC_FEATURE
<222>    (30)..(30)
<223>    K or R

<220>
<221>    PEPTIDE
<222>    (37)..(37)
<223>    epsilon K as a linker

```
<220>
<221>  PEPTIDE
<222>  (38)..(47)
<223>  LHRH

<400>  10

Thr Ala Lys Ser Lys Lys Phe Pro Ser Tyr Thr Ala Thr Tyr Gln Phe
1               5                   10                  15


Lys Ile Ser Ile Xaa Glu Ile Xaa Xaa Val Ile Val Xaa Xaa Ile Glu
            20                  25                  30


Thr Ile Leu Phe Lys Glu His Trp Ser Tyr Gly Leu Arg Pro Gly
        35                  40                  45


<210>  11
<211>  4
<212>  PRT
<213>  Homo sapiens


<220>
<221>  PEPTIDE
<222>  (1)..(4)
<223>  Spacer

<220>
<221>  PEPTIDE
<222>  (4)..(4)
<223>  epsilon K

<400>  11

Lys Lys Lys Lys
1


<210>  12
<211>  4
<212>  PRT
<213>  Homo sapiens


<220>
<221>  PEPTIDE
<222>  (1)..(1)
<223>  epsilon K

<220>
<221>  PEPTIDE
<222>  (1)..(4)
<223>  Spacer

<400>  12

Lys Lys Lys Lys
1
```